# FASCICULE DE BREVET EUROPEEN

(11) **EP 2 380 664 B1**
(45) Date de publication et mention de la délivrance du brevet: **05.12.2018**
(21) Numéro de dépôt: 11161711.4
(22) Date de dépôt: 08.04.2011
(51) Int. Cl.: B01J 31/18, C25B 11/04

(54) **Substrat fonctionnalisé et ses applications**
Funktionalisiertes Substrat und seine Anwendungen
Functionalized substrate and its applications

(30) Priorité: 20.04.2010 FR 1053019
(43) Date de publication de la demande: 26.10.2011
(73) Titulaire: COMMISSARIAT À L'ÉNERGIE ATOMIQUE ET AUX ÉNERGIES ALTERNATIVES, 75015 Paris (FR)
(72) Inventeur: Jacques, Pierre-André, 69280, SAINTE CONSORCE (FR); Aartero, Vincent, 38950, QUAIX-EN-CHARTREUSE (FR); Fontecave, Marc, 38330, SAINT ISMIER (FR); Leyris, Adeline, 07300, PLATS (FR); Matheron, Muriel, 73000, CHAMBERY (FR)
(74) Mandataire: Nony

(56) Documents cités:
- US-A- 3 584 022
- FONTECAVE MARC, ARTERO VINCENT: "Bioinspired catalysis at the crossroads between biology and chemistry: A remarkable example of an electrocatalytic material mimicking hydrogenases", COMPTE RENDUS CHIMIE, vol. xxx, 24 mars 2010 (2010-03-24), pages XXX-XXX, XP002613822, DOI: 10.1016/j.crci.2010.01.013
- JACQUES P -A; ARTERO V; PECAUT J; FONTECAVE M: "Cobalt and nickel diimine-dioxime complexes as molecular electrocatalysts for hydrogen evolution with low overvoltages", PROCEEDINGS OF THE NATIONAL ACADEMY OF SCIENCES OF THE UNITED STATES OF AMERICA, vol. 106, 8 décembre 2009 (2009-12-08), pages 20627-20632, XP002613823, ISSN: 0027-8424, DOI: 10.1073/pnas.0907775106

## Description

La présente invention concerne de nouveaux ligands et complexes catalytiques ainsi que des substrats fonctionnalisés par de telles entités.

### Arrière-plan

Les complexes métalliques sont bien connus pour leurs propriétés chimiques ou physicochimiques et valorisés à ce titre dans de nombreux domaines industriels dont, par exemple, les domaines de la catalyse, du magnétisme, de l'électronique moléculaire, de la luminescence, de l'environnement, de l'imagerie et de la thérapeutique.

D'une manière générale, ces complexes mettent en oeuvre un ou plusieurs métaux coordinés par un ou plusieurs ligands.

Parmi les ligands potentiels, les ligands tétradentate de type diimine-dioxime sont particulièrement appréciés du fait de leur aptitude à former des complexes stables avec des métaux de transition comme le rhodium (Rh), le fer (Fe), l'argent (Ag), le cuivre (Cu), le nickel (Ni) ou le cobalt (Co) (cf. Collman, J. P., Brauman, J. I., Madonik, A. M., Organometallics, 1986, 5, 310-322, Anderson, O. P., Perkins, C., Brito, K., Inorg. Chem. 1983, 22, 1267-1273, Eltayeb, M. A., Sulfab, Y., Polyhedron, 2007, 26,39-42, Stynes, Dennis V.; Singh, Kowsill; Ng, Betty; Wilshire, Susan; Inorg. Chim. Acta; Vol. 58; 1982, 58, 179 - 186). De manière générale, un ligand diimine-dioxime coordine les atomes métalliques en adoptant une conformation plane ou quasi-planaire. On dit alors qu'il coordine le métal dans un plan équatorial. D'autres positions de coordination sont alors laissées vacantes, que l'on qualifie de positions de coordination axiales.

En particulier, il est connu d'utiliser certains complexes diimine-dioxime comme catalyseurs de réactions de polymérisation du méthacrylate de méthyle (cf. Zangrando, E.; Trani, M.; Stabon, E.; Carfagna, C.; Milani, B.; Mestroni, G. Eur. J. Inorg. Chem. 2003, 2683-2692) ou d'électro-production d'hydrogène (cf. Jacques. P.-A.; Artero, V.; Pécaut, J.; Fontecave, M.; Proc. Natl. Acad. Sci. USA. 2009, 116, 20627-20632).

De tels complexes peuvent aussi être utilisés en imagerie où un complexe diimine-dioxime du ⁶⁴Cu a été envisagé comme composé radio pharmaceutique pour la tomographie par émission de positons (cf. Kiani, S.; Staples, R. J.; Treves, S. T.; Packard, A. B. Polyhedron 2009, 28, 775-781).

Il est aussi connu d'utiliser des complexes diimine-dioxime de cobalt comme mime de la vitamine B₁₂. Ces derniers sont capables de stabiliser soit une liaison cobalt-hydrure, soit une liaison cobalt-carbone pouvant se cliver de manière homolytique (cf. Gerli, A.; Sabat, M.; Marzilli, L. G.; J. Am. Chem. Soc. 1992, 114, 6711-6718).

L'immobilisation d'un ou plusieurs complexe(s) sur une surface possède de nombreux avantages (cf. Fontecave, M.; Artero, V.; C. R. Chimie, 2011, 14, 362-371). En effet, dans le cas où l'espèce chimique immobilisée est un catalyseur, son immobilisation rend la purification des produits finaux et le recyclage du catalyseur plus aisés par séparation mécanique du catalyseur immobilisé du milieu réactionnel. Dans d'autres cas, l'immobilisation permet de rendre un catalyseur plus stable et donc d'augmenter sa durée de vie et ses performances. Dans un contexte d'électro-catalyse, l'immobilisation d'un catalyseur sur une électrode permet également d'optimiser le flux des électrons depuis l'électrode vers le catalyseur et donc d'améliorer les densités de courant.

D'autres applications peuvent, par ailleurs, nécessiter de coupler un ligand ou un complexe avec une autre molécule, afin de pouvoir combiner les propriétés des deux partenaires. A ce titre, on peut, par exemple, citer la combinaison d'un ligand polydentate avec une entité fluorescente. Dans ce cas, la complexation du ligand modifie les propriétés de fluorescence du système, ce qui permet d'obtenir des capteurs qui renseignent sur la présence d'ions métalliques dans le milieu (cf. Valeur, B.; Leray, I. Coord. Chem. Rev. 2000, 205, 3-40).

Les ligands diimine-dioxime les plus étudiés sont ceux présentant une chaîne carbonée à deux ou trois atomes entre les deux atomes d'azote des fonctions imine. Cet espacement conduit, après complexation, à des métalla-cycles stables à 5 ou 6 chaînons. A titre illustratif de ce type de ligands, on peut citer ceux décrits dans la publication Dey et al; Synth. React. Inorg. Met.-Org. Chem.; 2004, 34; 1615 - 1634 qui portent une fonction hydroxyle parfois protégée.

Les complexes mettant en oeuvre ces ligands peuvent théoriquement se prêter à un couplage covalent avec un substrat selon plusieurs orientations, à savoir : l'orientation axiale (c'est-à-dire perpendiculairement au plan du ligand) et l'orientation équatoriale (c'est-à-dire dans le plan du ligand). Plus particulièrement, le couplage peut se faire via le ligand diimine-dioxime et on parle alors de couplage en orientation équatoriale (c'est-à-dire dans le plan du ligand). Alternativement, le couplage peut être effectué via un ligand axial et on parle alors de couplage en orientation axiale (c'est-à-dire généralement perpendiculairement au plan du ligand).

Les inventeurs ont constaté que, en comparaison avec une immobilisation en position axiale via un ligand généralement monodentate, le mode d'immobilisation en orientation équatoriale est plus stable car la nature polydentate du ligand diimine-dioxime permet de diminuer la probabilité de décrochage du complexe métallique.

Toutefois, les fonctions hydroxyle des ligands diimine-dioxime précités, précisément appropriées à un couplage en orientation équatoriale, s'avèrent en fait, sur le plan pratique, peu propices à un couplage covalent.

En effet, la formation d'un éther par la réaction de Williamson, nécessitant de produire l'alcoolate correspondant, se réalise dans des conditions très basiques que ne supporte pas le reste du ligand diimine-dioxime. Le couplage par estérification est également à proscrire du fait de l'équilibre de saponification qui met en cause la stabilité de l'édifice final. De plus, la chimie sur cette fonction hydroxyle entre en compétition avec les fonctions hydroxyle portées par les fonctions oximes, qui doivent garder leur intégrité pour ne pas modifier la sphère première de coordination du complexe métallique.

Ainsi, il existe un besoin de disposer de ligands diimine-dioxime ou de complexes métalliques correspondants aptes à être immobilisés de manière stable dans le temps sur une surface d'un substrat.

Il existe, en outre, un besoin de réaliser des assemblages moléculaires par couplage covalent de ligands diimine-dioxime ou de leurs complexes métalliques avec d'autres molécules.

La présente invention vise à résoudre tout ou partie des besoins précités.

### Résumé

Selon un premier de ses aspects, l'invention concerne un substrat en surface duquel est immobilisé au moins un motif de formule (I) : 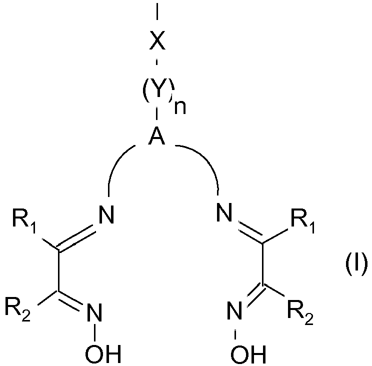 dans laquelle :
- R₁ et R₂, identiques ou différents, symbolisent un groupement choisi parmi : -H, un atome d'halogène, un éther en C₁ à C₂₀, une chaîne alkyle en C₁ à C₂₀, linéaire ou ramifiée éventuellement interrompue par un ou plusieurs hétéroatomes et/ou un ou plusieurs groupements aryles, ou un groupement aryle en C₅ à C₃₀ comprenant éventuellement un ou plusieurs hétéroatomes, ledit éther, ladite chaîne alkyle et ledit groupement aryle pouvant être substitués par au moins un des groupements suivants : -OH, -NH₂, -NO₂, -COOH, -CONH₂, un cycle triazole ou -COOZ₁ avec Z₁ représentant un groupe alkyle en C₁ à C₅, un groupe alcoxy en C₁ à C₅ ou un halogène,
- X est le produit d'une réaction de type « chimie-click », aza-Wittig, couplage peptidique ou le produit résultant de l'hydrogénation du produit obtenu à l'issue de ladite réaction d'aza-Wittig,
- Y est un bras espaceur,
- n est un nombre égal à 0 ou 1, et
- A:
   - représente une chaîne hydrocarbonée, saturée ou insaturée, en C₂ à C₃, ou
   - figure une double liaison C=C d'un cycle aromatique ou hétéroaromatique en C₅ à C₆ auquel cycle est lié Y, si n=1, ou X, si n=0.

Par « réaction de type « chimie-click » », on entend une réaction de couplage entre une fonction alcyne et une fonction azoture.

Par « réaction de type couplage peptidique », il faut comprendre une réaction conduisant à la formation d'un amide par couplage entre une fonction amine et une fonction carboxylique, ou un de ses dérivés comme une fonction halogénure d'acyle ou ester activé comme par exemple l'ester d'hydroxyphtalimide.

Par « substrat en surface duquel est immobilisé au moins un motif de formule (I) », il faut comprendre que le motif de formule (I) est présent en surface du substrat sous une forme accessible, c'est-à-dire propice à une interaction potentielle avec une ou plusieurs autre(s) molécule(s).

Comme il ressort de ce qui suit, cette immobilisation peut être de nature covalente ou non.

Selon une variante de réalisation de l'invention, le motif de structure (I) est avantageusement coordiné à au moins un métal de manière à former au moins un complexe métallique par exemple susceptible de manifester une activité catalytique.

Par métal on entend désigner au sens de l'invention les éléments suivants : Sc, Ti, V, Cr, Mn, Fe, Co, Ni, Cu, Zn, Y, Zr, Nb, Mo, Tc, Ru, Rh, Pd, Ag, Cd, Hf, Ta, W, Re, Os, Ir, Pt, Au, Hg, Ga, In, Sn, Tl, Pb, Bi, Po.

Comme il ressort de ce qui suit, les ligands considérés selon l'invention se prêtent avantageusement à une immobilisation sur un substrat du complexe métallique, formé en partie par au moins un desdits ligands, par l'intermédiaire d'une liaison en position équatoriale.

Comme mentionné plus haut, en comparaison avec une immobilisation en position axiale via un ligand généralement monodentate, le mode d'immobilisation en orientation équatoriale accessible selon l'invention s'avère avantageusement doté d'une stabilité accrue notamment du fait de la nature polydentate du ligand diimine-dioxime laquelle permet de réduire significativement la probabilité de décrochage du complexe métallique.

Selon un autre de ses aspects, l'invention concerne un ligand de formule (II): 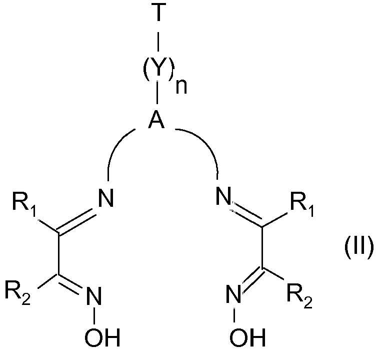 où :
- R₁, R₂, A, n et Y sont tels que définis ci-dessus, et
- T symbolise l'un des groupements suivants : N₃, NH₂ ou N=P(Ar)₃ pour lequel (Ar)₃ représente trois groupements aromatiques en C₆ à C₂₀, identiques ou non, éventuellement substitués, N=P(Ar)₃ étant de préférence N=PPh₃.

Selon encore un autre de ses aspects, l'invention concerne un ligand de formule (III) : 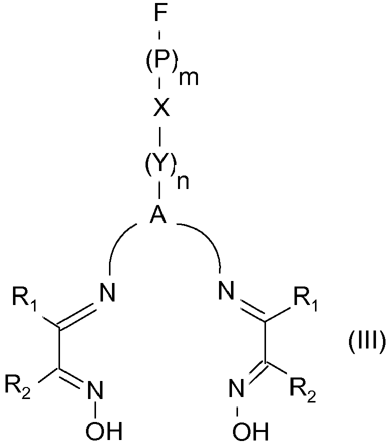 où :
- R₁, R₂, A, n, X et Y sont tels que définis ci-dessus,
- m est un nombre égal à 0 ou 1,
- P symbolise une chaîne hydrocarbonée en C₁ à C₂₀ comprenant le cas échéant un ou plusieurs hétéroatomes, éventuellement interrompue par un ou plusieurs groupements aryles, un éther en C₁ à C₂₀, un ester en C₁ à C₂₀ ou un groupement aryle en C₅ à C₃₀ comprenant éventuellement un ou plusieurs hétéroatomes intracycliques, P peut le cas échéant être substitué par au moins un des groupements suivants : -OH, -NH₂, -NO₂, -COOH, - CONH₂ ou -COOZ₂ avec Z₂ représentant un groupe alkyle en C₁ à C₅, un groupe alcoxy en C₁ à C₅ ou un halogène, et
- F symbolise une fonction réactive apte à réaliser une liaison covalente ou non-covalente et en particulier choisie parmi : -COOH, -NH₂, -N₃, -C≡CH, et un groupe silane choisi parmi : un groupe trihalogénosilane, un groupe trihydrogénosilane, un groupe trialcoxysilane -Si(OR₅)₃₋ avec R₅ représentant un groupe alkyle de 1 à 6 atomes de carbone, linéaire ou ramifié, un groupe phényle, un groupe triaminoalcoxysilane -Si(NR₆R₇)₃, avec R₆ et R₇ représentant indépendamment l'un de l'autre un groupe alkyle saturé de 1 à 6 atomes de carbone, linéaire ou ramifié, un groupe phényle ou un groupe organométallique.

Selon un autre de ses aspects, l'invention concerne une électrode comportant un substrat tel que défini ci-dessus.

Selon un autre de ses aspects, l'invention concerne un dispositif d'électrolyse comportant une électrode telle que définie ci-dessus.

Selon un autre de ses aspects, l'invention concerne un procédé de production de dihydrogène mettant en oeuvre un dispositif d'électrolyse tel que défini ci-dessus.

Selon un autre de ses aspects, l'invention concerne un procédé comportant au moins les étapes consistant à :
- disposer d'un substrat fonctionnalisé tel que défini ci-dessus ainsi que d'une source d'ions métalliques à analyser, et
- mettre en contact ledit substrat avec ladite source d'ions métalliques dans des conditions propices à la coordination des ions métalliques par le motif (I).

Ce procédé peut être un procédé de détection des ions métalliques, la coordination des ions métalliques par le motif (I) pouvant alors conduire à la modification d'au moins une propriété du substrat. Cette coordination peut, par exemple, entraîner la modification d'une propriété optique du substrat, notamment la modification de son spectre de fluorescence, ou la modification d'une propriété électrochimique du substrat, notamment la modification de son voltammogramme du fait de la présence des ions métalliques complexés.

Dans ce cas, le procédé peut, en outre, comprendre une étape consécutive destinée à caractériser le comportement optique ou électrochimique dudit substrat, notamment par référence à une valeur témoin.

Le procédé décrit ci-dessus peut également être un procédé d'extraction d'ions métalliques et, à l'issue de la complexation, le contact peut être interrompu entre le substrat et la solution d'ions métalliques afin d'extraire les ions métalliques complexés.

### Motif de formule (I)

Il est entendu que les exemples de réalisation donnés ci-dessous pour R₁, R₂, A, Y s'appliquent aux motifs de formule (I) ainsi qu'aux ligands de formules (II) et (III).

Par ailleurs, les exemples de réalisation donnés ci-dessous pour X s'appliquent aux motifs de formule (I) ainsi qu'aux ligands de formule (III).

De préférence, le motif de formule (I) peut présenter l'une des formules suivantes : 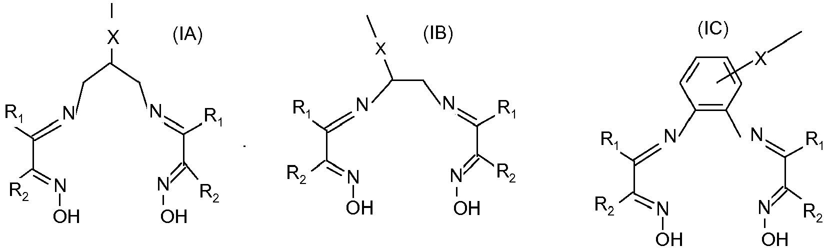 où R₁, R₂ et X peuvent être tels que définis ci-dessus.

Dans une variante particulièrement préférée, le motif de formule (I) peut être de formule : 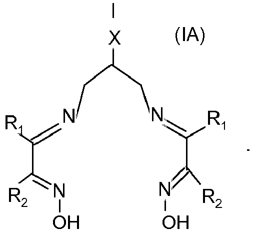 où R₁, R₂ et X peuvent être tels que définis ci-dessus.

### Définition de X

- X peut, de préférence, être de formule : 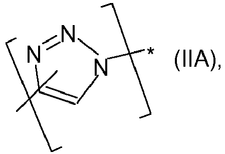 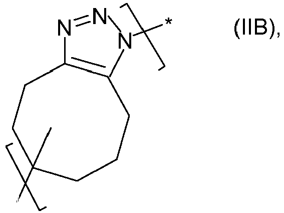 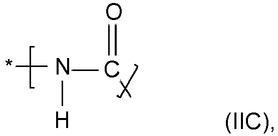 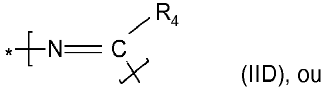 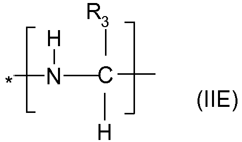 dans lesquelles :
   - * symbolise une liaison vers Y si n=1 ou vers A si n=0, et
   - R₃ et R₄ sont choisis parmi : -H, une chaîne alkyle en C₁ à C₂₀, un éther en C₁ à C₂₀, un ester en C₁ à C₂₀ ou un groupement aryle en C₅ à C₃₀, R₃ et R₄ étant le cas échéant substitués par au moins un des groupements suivants : -OH, -NH₂, -COOH ou -CONH₂.

Lorsque X est de formule (IIE), le motif (I) est, avantageusement, coordiné par au moins un métal.

Dans une variante encore plus préférée, X peut être de formule : 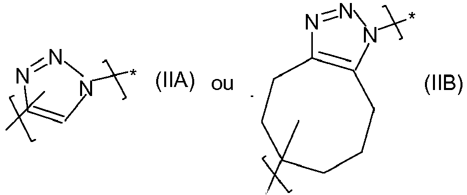 où * est tel que défini ci-dessus.

### Définition de Y

Y est un bras espaceur dont la fonction est de lier X à A lorsque n est égal à 1.
- Lorsque n est égal à 1, Y peut, par exemple, être choisi parmi une chaîne alkyle en C₁ à C₂₀, linéaire ou ramifiée éventuellement interrompue par un ou plusieurs hétéroatomes et/ou un ou plusieurs groupements aryles, un éther en C₁ à C₂₀, un ester en C₁ à C₂₀ ou un groupement aryle en C₅ à C₃₀ comprenant éventuellement un ou plusieurs hétéroatomes, Y peut le cas échéant être substitué par au moins un des groupements suivants : -OH, -NH₂, -NO₂, -COOH, -CONH₂ ou -COOZ₂ avec Z₂ représentant un groupe alkyle en C₁ à C₅, un halogène ou un groupe alcoxy en C₁ à C₅.

### Définition de A

- Pour sa part, A peut, de préférence, être une chaîne hydrocarbonée saturée en C₂ à C₃.

Dans une variante encore plus préférée, A peut être une chaîne hydrocarbonée saturée en C₃.

### Définition de R₁ et R₂

- Les deux motifs R₁ peuvent être identiques ou différents. De même, les deux motifs R₂ peuvent être identiques ou différents.
- R₁ et R₂ peuvent, par exemple, être identiques ou différents et choisis parmi : -H ou une chaîne alkyle en C₁ à C₂₀ éventuellement substituée par l'un au moins des groupements suivants : - OH, -NH₂, -COOH, -CONH₂ ou un cycle triazole.

R₁ et R₂ peuvent, par exemple, être identiques.

R₁ et R₂ peuvent, par exemple, être tous deux choisis parmi les chaînes alkyles en C₁ à C₂₀ éventuellement substituées.

R₁ et R₂ peuvent, par exemple, être tous deux des groupements méthyles éventuellement substitués.

Le motif de formule (I), coordiné à un métal ou non, peut être directement lié au substrat.

Dans ce cas, le procédé d'immobilisation dépend de la nature de l' ou des extrémité(s) réactive(s) du substrat, d'une part, et de celle du dérivé diimine-dioxime d'autre part.

Au regard de la nature du substrat, le motif (I), coordiné à un métal ou non, peut être lié de manière covalente ou non covalente au substrat.

En variante, le motif de formule (I) peut être lié au substrat via un bras de liaison, dit encore L.

### Définition du bras de liaison

Dans ce cas, le procédé d'immobilisation dépend de la nature de l' ou des extrémité(s) réactive(s) du substrat, d'une part, et de celle(s) du bras de liaison d'autre part.

Le bras de liaison peut être immobilisé sur le substrat de manière à assurer un bon transfert électronique entre le substrat et le motif (I) éventuellement coordiné par un métal.

Le bras de liaison peut être lié au substrat de manière covalente ou non-covalente.

L'interaction non-covalente peut, de préférence, être une interaction électrostatique ou une interaction π-π.

En outre, le bras de liaison peut être lié à un ou plusieurs motif(s) de formule (I).

Le motif de formule (I), coordiné à un métal ou non, peut, par exemple, être lié au bras de liaison de manière covalente.

Pour des raisons évidentes, le choix du bras de liaison et plus particulièrement des motifs présents à ses extrémités est lié à la nature du substrat ainsi qu'à la fonction réactive présente sur le dérivé diimine-dioxime à immobiliser.

Il est à la portée de l'homme du métier d'ajuster la nature chimique des deux extrémités réactives du bras de liaison afin de permettre la création des liaisons au niveau respectivement du substrat et du motif (I).

Dans le cas où le motif de formule (I) est lié au substrat via un bras de liaison, un motif de formule (I') suivante peut être immobilisé en surface du substrat. 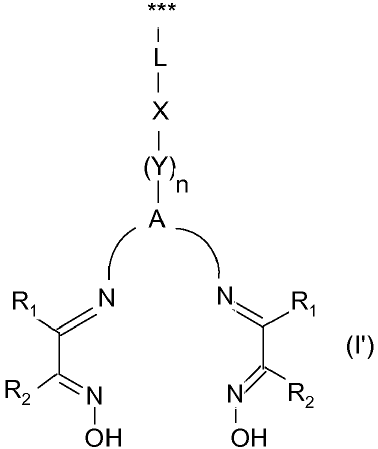 où :
- R₁, R₂, A, Y, n et X sont tels que définis ci-dessus,
- L symbolise le bras de liaison, et
- *** symbolise une liaison vers le substrat.

La nature chimique du bras de liaison L n'est pas déterminante tant que ce dernier ne perturbe pas outre mesure les propriétés, notamment en termes de réactivité ou de catalyse, du motif (I) et de ses complexes.

A titre illustratif, le bras de liaison peut, par exemple, être de formule (IV) ou (V): 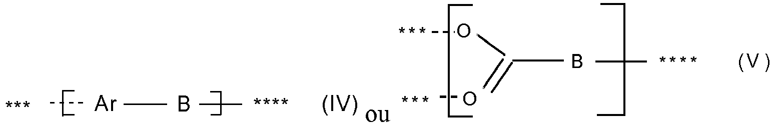 où :
- *** est tel que défini ci-dessus et **** symbolise une liaison vers X,
- Ar symbolise un groupement aryle en C₆ à C₃₀ éventuellement substitué par au moins un des groupements suivants : -OH, -NH₂, -COOH, -CONH₂ ou un cycle triazole, Ar étant apte à établir une interaction figurée par ----, covalente ou non covalente avec le substrat, et
- B est un groupement divalent hydrocarboné éventuellement interrompu par un motif: -COO- ou -CONH- et dont l'extrémité liée à X est formée d'un motif résultant d'un couplage entre deux fonctions réactives, tel que, par exemple, un motif amide, ester ou éther.

B peut, par exemple, comporter une extrémité liée à X figurée par un motif éther.

Lorsque le bras de liaison est de formule (IV), ce dernier peut, par exemple, être lié au substrat de manière covalente.

Lorsque le bras de liaison est de formule (IV), la liaison entre ce dernier et le substrat peut, par exemple, être obtenue par une réaction de réduction de sels d'aryldiazonium.

Lorsque le bras de liaison est de formule (V), ce dernier peut être lié au substrat de manière non-covalente, par exemple par une interaction électrostatique.

Il est manifeste que la préparation d'un substrat conforme à la présente invention relève des compétences de l'homme du métier.

Selon un exemple de réalisation, un tel substrat peut être obtenu par un procédé comportant au moins les étapes consistant à :
a) lier le bras de liaison au substrat, et
b) lier le bras de liaison au motif (I), le motif (I) étant coordiné ou non à un métal,
l'étape a) ayant lieu avant ou après l'étape b).

### Complexes métalliques

Comme mentionné plus haut, le motif de structure (I) peut être coordiné à au moins un métal de manière à former au moins un complexe métallique.

Comme précisé ci-dessus, le métal est choisi parmi : Sc, Ti, V, Cr, Mn, Fe, Co, Ni, Cu, Zn, Y, Zr, Nb, Mo, Tc, Ru, Rh, Pd, Ag, Cd, Hf, Ta, W, Re, Os, Ir, Pt, Au, Hg, Ga, In, Sn, Tl, Pb, Bi ou Po.

Le métal peut, de préférence, être un métal de transition de la classification périodique.

Dans une variante particulièrement préférée, le métal est choisi parmi le nickel (Ni) et le cobalt (Co).

Dans une variante particulièrement préférée, le complexe métallique formé avec ledit motif de formule (I) peut présenter l'une des formules suivantes : 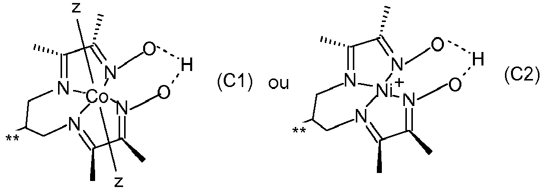 où Z désigne un atome d'halogène, de préférence le chlore ou le brome, et ** symbolise la liaison vers X via Y si n=1.

### Substrat

Au sens de l'invention, le substrat peut comporter, par exemple consister en, un matériau ou consister en une ou plusieurs molécule(s) d'intérêt.

Dans la variante où le substrat comporte, voire consiste en, un matériau, ce dernier peut comporter un matériau conducteur ou semi-conducteur ayant une surface spécifique élevée.

Ce matériau conducteur ou semi-conducteur avec une surface spécifique élevée peut être déposé sur un support conducteur afin de former une électrode ayant une surface spécifique élevée.

Le support conducteur peut comporter, par exemple constituer en, de l'ITO (« Indium Tin Oxide »), ou un autre oxyde transparent conducteur comme F:SnO₂ (« Fluorine Tin Oxide ») ou Al: ZnO (ZnO dopé à l'aluminium), de l'acier inoxydable, du fer, du cuivre, du nickel, du cobalt, de l'aluminium, de l'or, du diamant dopé, du titane, du laiton ou du carbone, par exemple du graphite. Le support conducteur peut, de préférence, comporter, par exemple consister en, de l'ITO ou du graphite.

Lorsque le substrat comporte, voire consiste en, un matériau, celui-ci peut être choisi parmi:
- les oxydes semi-conducteurs, par exemple : ITO (« Indium Tin Oxide »), FSnO₂ (« Fluorine Tin Oxide »), ZnO, Al:ZnO (ZnO dopé à l'aluminium), Na:ZnO (ZnO dopé au sodium), NiO ou TiO₂, ou
- les matériaux conducteurs ou semi-conducteurs par exemple :
   - les métaux par exemple le silicium, éventuellement dopé, le laiton, l'acier inoxydable, le fer, le cuivre, le nickel, le cobalt, l'aluminium, l'argent, l'or, le titane,
   - les oxydes métalliques, ou
   - les matériaux carbonés par exemple les nanotubes de carbone mono ou multiparois, le noir de carbone, les nanoparticules fullérèniques, le graphite, le carbone vitreux, le graphène ou le diamant dopé.

Le matériau constituant au moins en partie le substrat peut être solide ou semi-solide.

Le matériau constituant au moins en partie le substrat peut être nanostructuré ou non.

Par « matériau nanostructuré », il faut comprendre soit que le matériau possède une rugosité de surface importante et/ou une surface spécifique élevée, soit qu'il peut être formé en tout ou partie de particules, par exemple de diamètre compris entre 2 nm et 10 µm, et en particulier entre 5 nm et 300 nm.

Comme mentionné plus haut, le substrat peut, en variante, être constitué d'une ou plusieurs molécule(s) d'intérêt notamment une ou plusieurs molécule(s) biologique(s), par exemple choisie(s) parmi : les marqueurs, les colorants, les fluorophores (tels que par exemple les coumarines, fluorescéine, fluorescines modifiées, rhodanines, iguanines, boron-dipynométhène, oxazines et autres), les molécules radiomarquées, les agents de contraste (rayons X, IRM), les toxines (y compris les cytotoxines), les linkers, les actifs thérapeutiques, cosmétiques, phytosanitaires, les membres d'une paire liante spécifique, les peptides, les aminoacides et les résidus d'acides aminés, les polypeptides (y compris les peptides et les protéines), les sucres et les résidus de sucres, les oligonucléotides, les brins d'ADN, d'ARN, les anticorps, les antigènes, des composés présentant une isomérie cis-trans tels que les dérivés des diaryléthylènes, des spiropyrannes, des spiroxazines, des fulgides ou de l'azobenzène (dont la présence sur une surface est particulièrement utile pour la fabrication d'interrupteurs moléculaires photocontrôlés).

La molécule d'intérêt peut, de préférence, être un fluorophore.

Selon une variante préférée, la molécule d'intérêt est une molécule biologique.

Les biomolécules d'intérêt peuvent être d'origine naturelle ou produites de manière synthétique ou recombinante et peuvent être isolées, purifiées.

Lorsque la molécule d'intérêt est un polypeptide, le polypeptide peut être composé d'aminoacides D,L ou des deux, et peut en outre être modifié, que ce soit de manière naturelle, synthétique ou recombinante, afin d'y inclure d'autres groupements. Par exemple, le polypeptide cible peut être une lipoprotéine, une glycoprotéine, ou tout autre protéine modifiée.

Selon une autre variante de réalisation, la molécule d'intérêt peut être un agent contrastant.

### Description des figures

- la figure 1 illustre un schéma de synthèse d'un ligand diimine-dioxime selon l'invention,
- les figures 2 et 3 représentent des analyses de voltammétrie cyclique de complexes métalliques,
- les figures 4A et 5 représentent des voltammogrammes cycliques enregistrés sur un substrat selon l'invention, et
- la figure 4B est un graphique tiré des résultats illustrés en figure 4A.

### Exemples

Les spectres RMN décrits dans les exemples ont été enregistrés à température ambiante dans des tubes de 5 mm de diamètre sur un spectrophotomètre Bruker AC 300. Les pics résiduels du solvant incomplètement deuteré ont été utilisés comme références internes.

Les spectres de masse (ionisation par électro-spray) décrits dans les exemples ont été mesurés sur un appareil Finnigan LCQ thermoquest de type « ion-trap ».

Les mesures électrochimiques décrites dans les exemples ont été réalisées de manière classique avec un montage à trois électrodes et un potentiostat EG&G, modèle 273A. L'électrode auxiliaire est un fil de platine, l'électrode de référence est une électrode Ag/AgCl, KCl 3M. L'électrode de travail est soit une électrode de carbone vitreux, soit une lame d'ITO fonctionnalisée, soit une électrode à base de nanotubes de carbone décrite dans: Le Goff et al., M. Science 2009, 326, 1384-1387. Les potentiels sont parfois donnés par rapport au couple ferricinium/ferrocène (Fc⁺/Fc) qui a été remesuré en ajoutant du ferrocène dans l'électrolyte en fin de manipulation. L'électrolyte est une solution 0.1 M de tétrafluoroborate de tétrabutylammonium dans l'acétonitrile.

### Exemple 1 : Synthèse d'un ligand de formule (II) ayant une fonction d'accroche T de type azoture

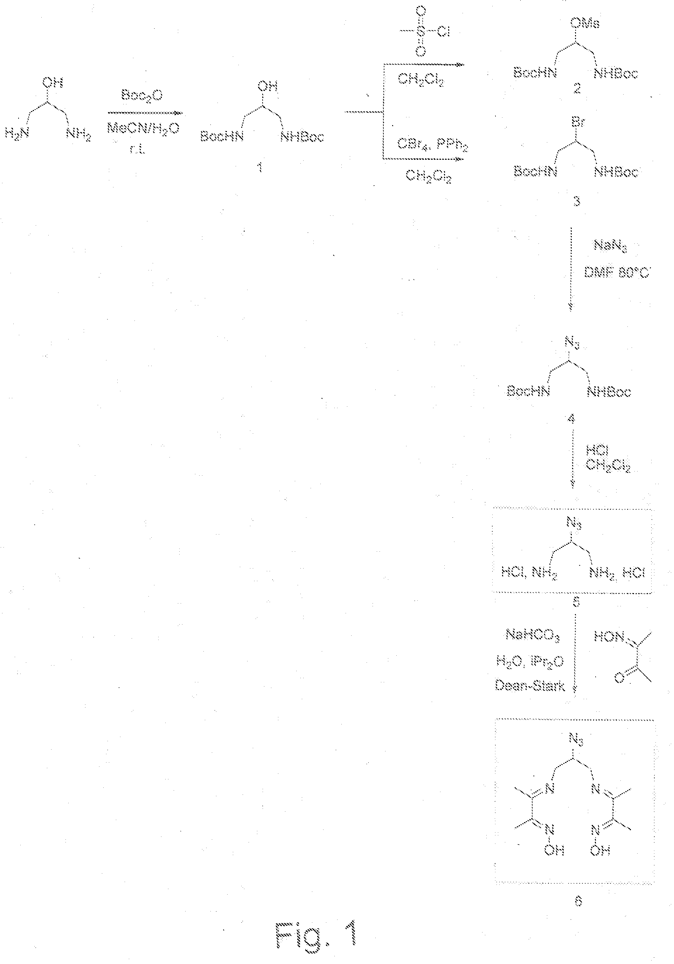

La figure 1 résume la voie de synthèse mise en oeuvre pour obtenir le ligand diimine-dioxime **6** renfermant un groupe T azoture, un groupe A de type chaîne hydrocarbonée saturée en C₃ et des substituants R₁ = Me et R₂ = Me.

### Synthèse du N,N'-Di-t-boc-2-hydroxy-1,3-diaminopropane (1)

A une solution de 2-hydroxy-1,3-diaminopropane (5 g, 55 mmol) dans 100 ml d'un mélange MeCN/H₂O (5/5) est ajoutée une solution di-t-butylcarbonate (26 ml, 113 mmol) dans MeCN à 0 °C. On laisse ensuite la température du mélange remonter à température ambiante et le mélange est maintenu sous agitation pendant 18 h. La solution est ensuite extraite au CH₂Cl₂ (200ml) et la phase organique est lavée avec une solution aqueuse saturée de NaHCO₃ (3x50ml), séchée sur MgSO₄, puis concentrée sous pression réduite. Un solide blanc (14,7 g, 51 mmol) est collecté.
¹H NMR (300 MHz, CDCl₃): δ (ppm) 5,34 (s, 2H, NH); 4.00 (bs, 1H, OH), 3,72 (m, 1H, C(OH)H) ; 3,16 (m, 4H, CH₂), 1.40 (s, 18H, ^{t}Boc).

### Synthèse du N,N'-Di-t-boc-2-methanesulfonyl-1,3-diaminopropane (2)

A une solution de **1** (4 g, 14 mmol) dans CH₂Cl₂ (100 ml) sous Ar contenant NEt₃ (2,9 ml, 20 mmol) refroidie à 0 °C est ajouté goutte à goutte du chlorure de méthanesulfonyle (2,15 ml, 28 mmol). On laisse ensuite la température du mélange remonter à température ambiante et le mélange est maintenu sous agitation pendant 2 h. Ensuite, 2 autres équivalents de chlorure de methanesulfonyle (2,15 ml, 28 mmol) sont ajoutés au mélange préalablement refroidi à 0 °C. Le mélange est agité à température ambiante pendant 18 h puis est extrait avec une solution aqueuse saturée de NaHCO₃ (5x50 ml), la phase organique est séchée sur MgSO₄ et est concentrée sous pression réduite. Un solide blanc (4.8 g, 13 mmol) est collecté.
¹H NMR (300 MHz, CDCl₃): δ (ppm) 5,18 (bs, 2H, NH); 4,68 (m, 1H, C(OMs)H); 3,49 (m, 2H, CH₂); 3,33 (m, 2H, CH₂); 3,11 (s, 3H, OMs); 1,46 (s, 18H, *^{t}*Boc).

### Synthèse du N,N'-Di-t-boc-2-bromo-1,3-diaminopropane (3)

A une solution de **1** (2,9 g, 9.3 mmol) dans CH₂Cl₂ (30 ml) contenant PPh₃ (2,93 g, 1.1 mmol) refroidie à 0 °C est ajouté CBr₄ (3,7g, 11.1 mmol) en solution dans CH₂Cl₂ (20ml). La solution est agitée 2 h à 0 °C puis 3 h à température ambiante. Le solvant est évaporé sous pression réduite et le produit est purifié par chromatographie sur gel de silice. Un premier lavage au pentane permet d'éliminer des impuretés puis le produit est élué avec un mélange pentane/AcOEt 7/3. Un solide blanc est collecté (2,2 g, 6,3 mmol).
¹H NMR (300 MHz, CDCl₃): δ (ppm) 5,32 (bs, 2H, NH); 4.11 (m, 1H, CHBr), 3,74 (m, 2H, CH₂); 3,27 (m, 2H, CH₂); 1,47 (s, 18H, *^{t}*Boc).

### Synthèse du N,N'-Di-t-boc-2-azide-1,3-diaminopropane (4)

A une solution de 2 (5 g, 14 mmol) dans du DMF sec (20 ml) est ajouté NaN₃ (3,54 g, 54 mmol) en suspension dans du DMF sec (10 ml). Le mélange est chauffé à 80 °C pendant 18h. Ensuite, de l'eau (100 ml) et du CH₂Cl₂ (100ml) sont ajoutés au mélange puis la phase organique est lavée avec de l'eau (3x50 ml). La phase organique est séchée sur MgSO₄ et est concentrée sous vide. Une huile jaune (3.2 g, 10 mmol) est collectée.
¹H NMR (300 MHz, CDCl₃): δ (ppm) 5,07 (bs, 2H, NH); 3,66 (m, 1H, CHN₃); 3,38 (m, 2H, CH₂); 3,16 (m, 2H, CH₂); 1,47 (s, 18H, *^{t}*Boc).
Le même protocole peut être appliqué en utilisant **3** au lieu de **2** comme produit de départ.

### Synthèse du 2-azide-1,3-diaminopropane hydrochloride salt (5)

HCl gazeux est mis à barboter dans une solution de **4** (2g, 6,3 mmol) dans CH₂Cl₂ à 0 °C pendant 2 h. Le précipité blanc est ensuite filtré et lavé avec Et₂O puis séché sous vide (1,13 g, 6,0 mmol).
¹H NMR (300 MHz, D₂O): δ (ppm) 4,16 (m, 1H, CHN₃), 3.27 (m, 2H, CH₂); 3,04 (m, 2H, CH₂).

### Synthèse du N²,N²'-2-azidepropanediylbis(2,3-butadione-2-imine-3-oxime) (6)

Une solution de **5** (200 mg, 1,1 mmol), 2,3-butadione-monoxime (216 mg, 2,2 mmol) et NaHCO₃ (180 mg, 2,2 mmol) dans un mélange biphasique d'eau (5ml) et de *ⁱ*Pr₂O (20 mL) est chauffée à reflux pendant 18 h en utilisant un appareil de Dean-Stark. Ensuite, le mélange est filtré à chaud et le précipité lavé avec du CH₂Cl₂. Le solvant est évaporé, le solide est dissous dans un minimum d'AcOEt et est précipité par ajout de pentane, filtré et séché sous vide.
¹H NMR (300 MHz, DMSO): δ (ppm) 11,54 (s, 2H, NOH); 4,02 (m, 1H, CHN₃); 3,62 (m, 4H, CH₂); 2,02 (s, 3H, CH₃); 1,94 (s, 3H, CH₃).

Si la réaction n'est pas totale, on reprend le tout dans l'EtOH distillé, de l'orthoformiate d'éthyle est ajouté au mélange qui est ensuite chauffé au reflux pendant 8 h. Puis le solvant est évaporé et le produit est précipité dans le pentane.

### Exemple 2

Cet exemple concerne la préparation des complexes dont les formules sont données ci-dessous :

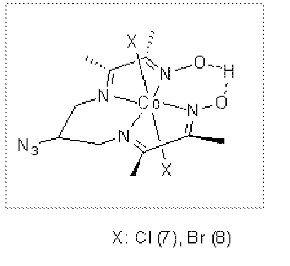

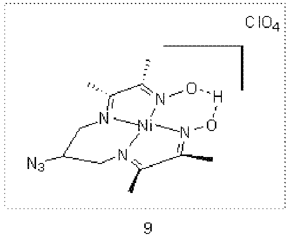

Des métaux de transition tel que le cobalt ou le nickel peuvent être complexés par le ligand tétradentate fonctionnalisé obtenu à l'exemple 1.

### Synthèse du complexe [Co(DO)(DOH)N₃pnCl₂] (7)

A une solution de **6** (50 mg, 0,18 mmol) dans l'acétone (20ml) est ajoutée une solution de CoCl_{2,}, 6H₂O (42.5 mg, 0,18 mmol) dans l'acétone (5ml). Sous barbotage d'air, le mélange marron devient vert. Après 30 min, le solvant est évaporé et un solide vert est collecté. Il est repris d'abord à l'acétone puis au dichlorométhane pour conduire à 7 (92 mg, 0,18 mmol).
¹H NMR (300 MHz, acétone *d⁶*): δ (ppm) 19,39 (s, 1H, OHO); 4,56 (m, 3H, CH₂, CHN₃); 3,90 (t, 13.2 Hz, 2H, CH₂); 2,77 (s, 6H, CH₃); 2,55 (s, 6H, CH₃).

### Synthèse du complexe [Co(DO)(DOH)N₃pnBr₂] (8)

La même procédure que pour 7 est appliquée en utilisant CoBr₂, 6H₂O.
¹H NMR (300 MHz, acétone *d⁶*): δ (ppm) 19,45 (s, 1H, OHO); 4,70 (m, 3H, CH₂, CHN₃); 3,89 (m, 2H, CH₂); 2,80 (s, 6H, CH₃); 2,57 (s, 6H, CH₃).

### Synthèse du complexe [Ni(DO)(DOH)N₃pn]ClO₄ (9)

A une solution de **6** (50 mg, 0,18 mmol) dans MeOH (20 ml) est ajouté un excès de NiCl₂ (60 mg, 0,25 mmol) en solution dans le MeOH (5 ml). Après 30 min, le mélange est filtré, le filtrat est concentré sous pression réduite et est repris dans l'eau. Le complexe de Ni est précipité par addition d'une solution de NaClO₄. Le solide orange est récupéré par filtration (35 mg, 0,08 mmol).
¹H NMR (300 MHz, acétone *d⁶*): δ (ppm) 18,28 (s, 1H, OHO); 4,63 (m, 1H, CHN₃); 3,90 (m, 2H, CH₂); 3,70 (d, J: 15,9 Hz, 2H, CH₂); 2,47 (s, 6H, CH₃); 2,21 (s, 6H, CH₃). ¹³CH NMR (75 MHz, acetone *d⁶*): 179,3 (CNOH); 156,2 (CN); 58,6 (CH); 50,6 (CH₂); 16,5 (CH₃); 11,5 (CH₃).

Le tableau 1 ci-dessous présente les paramètres cristallographiques des complexes (7) et (9) obtenus.

**Tableau 1**

| | | |
|---|---|---|
| Composé | [Co(DO)(DOH)N₃pnCl₂] | [Ni(DO)(DOH)N₃pn](ClO₄) |
| Formule | C₁₁H₁₈Cl₂CoN₇O₂ | C₁₁H₁₈ClN₇NiO₆ |
| Masse moléculaire | 425,15 | 338,48 |
| Couleur | Vert | Marron |
| Taille du cristal | 0,93 x 0,73 x 0,33 mm | 0,66 x 0,41 x 0,29 mm |
| Système cristallin | Monoclinique | Orthorhombique |
| Groupe d'espace | *P1* 2₁/*n 1* | *P* 2₁ 2₁ 2₁ |
| *a* [Å] | 10,0677 (19) | 6,8991 (2) |
| *b* [Å] | 14,6157(3) | 11,7274 (3) |
| *c* [Å] | 11,4658 (2) | 20,7645 (6) |
| *α* [°] | 90 | 90 |
| *β* [°] | 97,5274 (18) | 90 |
| *γ* [°] | 90 | 90 |
| *V* [Å]³ | 1672,62 (5) | 1680,02 (9) |
| Z | 4 | 4 |
| ρ_{calcd} [mg m⁻³] | 1,688 | 1,734 |
| µ [mm⁻¹] | 1,372 | 1,361 |
| Réflexions collectées | 9728 | 23236 |
| Réflexions uniques (Rᵢₙₜ) | 5026 (Rᵢₙₜ=0.0187) | 12364 (Rᵢₙₜ=0,0291) |
| Réflexions observées [*I*>2σ(*I*)] | 3785 | 6843 |
| Nombre de paramètres affinés | 273 | 278 |
| Indices R (toutes réflexions) | R1 =0,0455, wR2 =0,0771R1 =0,0987, wR2 =0,1394 | |
| Indices R (réflexions observées)R1 | =0,0307, wR2 =0,0744R1 =0,0547, wR2 =0,1296 | |
| | | |
| Qualité de l'affinement S | 1,015 | 0,961 |
| Δρ (max/min) [e Å⁻³] | 0,595 et -0,370 | 0,957 et -1,047 |

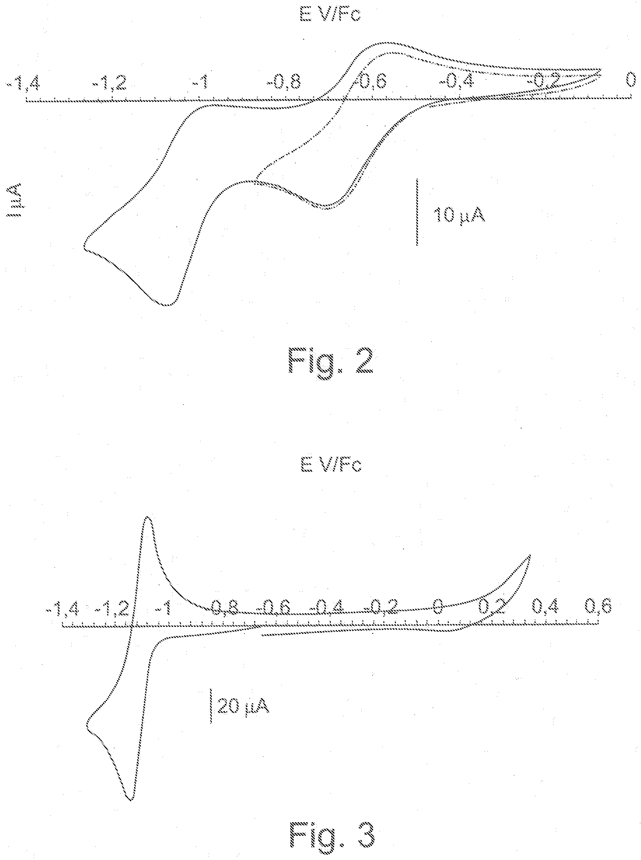

Les figures 2 et 3 présentent les voltammogrammes cycliques des composés 7 et **9** enregistrés à 100 mV.s⁻¹ dans l'acétonitrile sur électrode de carbone vitreux (solution de complexe à 1 mM dans l'acétonitrile et à 0,1 M en tétrafluoroborate de térabutylammonium).

Les potentiels électrochimiques sont donnés par rapport au couple ferrocène-ferricinium.

### Exemple 3 : Synthèse d'un complexe métallique mettant en oeuvre un ligand de formule (II) ayant un groupement T de type -N=PPh₃

La réaction du complexe **9** avec la triphénylphosphine dans le THF conduit à la formation du composé phosphine-imine **10a** qui a été caractérisé par RMN ¹H et par spectrométrie de masse.

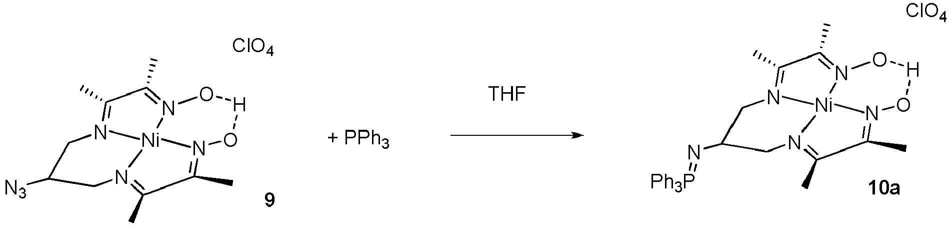

Le protocole de synthèse du complexe Ni(DO)(DOH)(CH₂CH(-N=PPh₃)CH₂)]ClO₄ **10a** est le suivant : dans un ballon, 35 mg du complexe de nickel **9** et 21 mg de triphénylphosphine sont dissous dans 10 ml de THF (non distillé). Après une 24 heures d'agitation, le THF est évaporé et le résidu est lavé à l'eau puis séché à l'air.
¹H NMR (300 MHz, acétone *d⁶*): δ (ppm) 2,1 (s, 6H); 2,8 (s, 6H); 3,55-3,65 (m, 4H); 4,21 (m, 1H), 7,71-7,94 (m, 15H); 18,33 (s, 1H).
ESI-MS : m/z : 572,3.

### Exemple 4 : Synthèse d'un complexe métallique mettant en oeuvre un ligand de formule (II) ayant un groupement T de type -NH₂

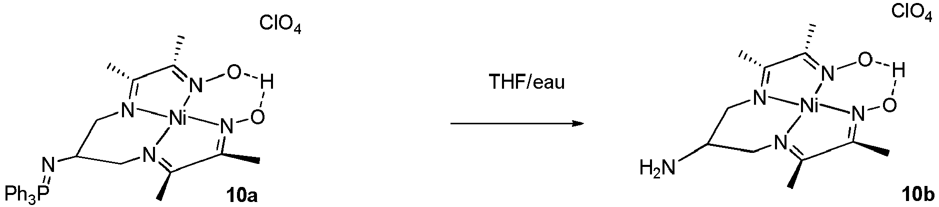

L'hydrolyse de **10a** par station prolongée dans un mélange THF/eau conduit au dérivé amino correspondant **10b.**

Le protocole de synthèse du complexe Ni(DO)(DOH) (CH₂CH(-NH₂)CH₂)]ClO₄ **10b** est le suivant : dans un ballon, 40 mg du complexe de nickel **10a** sont dissous dans 10 mL de THF (non distillé) et 4 mL d'eau desionisée. Après 24 heures d'agitation, le THF est évaporé et le résidu est lavé à l'eau puis séché à l'air.

### Exemple 5 : Synthèse de complexes métalliques mettant en oeuvre des ligands de formule (III)

Les schémas des synthèses correspondant à cet exemple sont présentés ci-dessous.

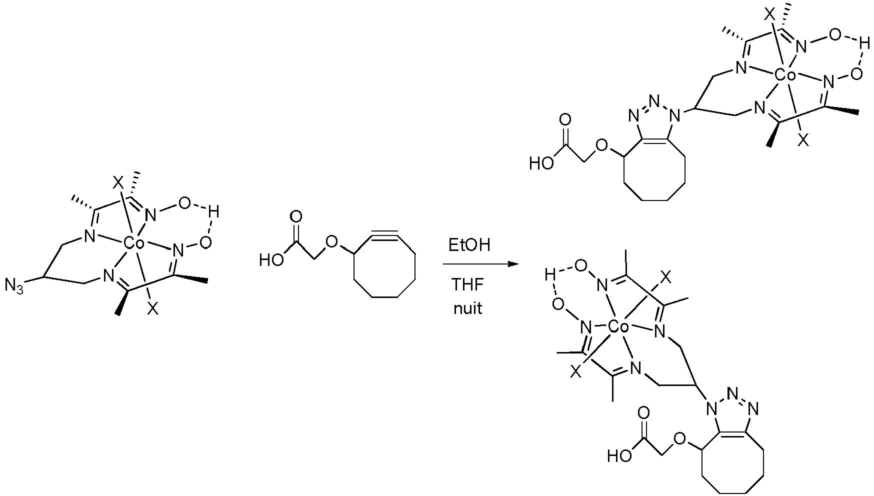

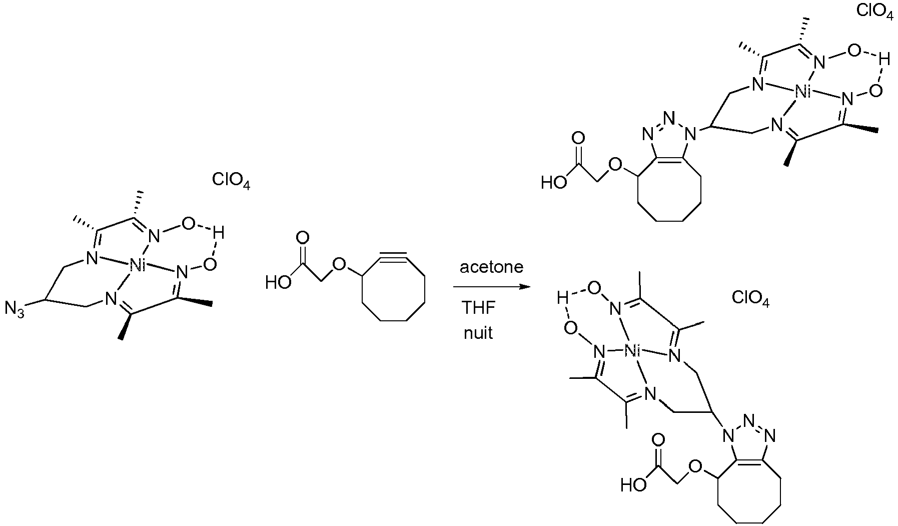

Le cyclooctyne **14** a été synthétisé selon un mode opératoire décrit dans la publication : Agard, N. J.; Baskin, J. M. ; Prescher, J. A. ; Lo, A.; Bertozzi, C. R.; A comparative study of bioorthogonal reactions with azides. Acs Chemical Biology 2006, 1, (10), 644-648 et la demande de brevet US 2006/0110782.

Dans cette approche, deux isomères sont formés au cours de la réaction. Les schémas ci-dessus montrent les produits de la réaction des complexes **7**, **8** et **9** avec le cyclooctyne **14**.

Le protocole correspondant au schéma supérieur est le suivant : à une solution de 12 mg (6,6 10⁻⁵ mol; 1 eq.) de l'acide cyclooct-1-yn-3-glycolique **14** dans le THF est ajoutée une solution de 27 mg (6,6 10⁻⁵ mol; 1 eq.) de complexe de cobalt 7 dans l'éthanol. Le mélange réactionnel est agité 18 heures à température ambiante. Les solvants organiques sont ensuite évaporés sous pression réduite. Le produit est purifié sur colonne d'exclusion de taille type Sephadex.

Le protocole correspondant au schéma inférieur est le suivant : à une solution de 12 mg (6,6 10⁻⁵ mol; 1 eq.) de l'acide cyclooct-1-yn-3-glycolique **14** dans THF est ajoutée une solution de 29 mg (6,6 10⁻⁵ mol; 1 eq.) du complexe de nickel **9** dissous dans l'acétone. Le mélange réactionnel est agité 18 heures à température ambiante. Le milieu réactionnel est concentré sous pression réduite puis séché à l'air. Le produit est purifié sur colonne d'exclusion de taille type Sephadex.

### Exemple 6: Immobilisation par cycloaddition de Huisgen sur une surface d'ITO d'un complexe de coordination

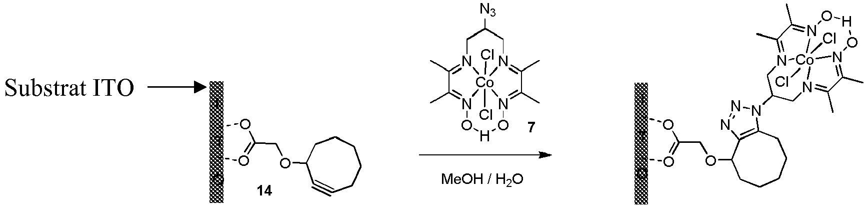

Le couplage de Huisgen peut être réalisé sur une surface d'ITO préalablement fonctionnalisée avec une fonction cyclooctyne.

Les lames d'ITO utilisées proviennent de Präzisions Glas & Optik GmbH en Allemagne, référence CEC005S, résistivité </= 5 Ohms/sq telle que définie de manière connue pour les couches minces conductrices. L'ITO est déposé sur un substrat de verre, d'épaisseur 1,1 mm et passivé par une fine couche de SiO₂.

### Préparation de surface des lames d'ITO

Les lames sont lavées par le biais d'ultrasons pendant 5 minutes dans l'acétone puis sont activées aux ultrasons pendant 30 minutes dans une solution de NaOH (0,1 M) dans un mélange éthanol/eau (43/57).

### Les lames sont ensuite rincées à l'eau déionisée et séchées sous flux d'argon.

Immédiatement après, les lames subissent un traitement plasma à oxygène. L'appareil est réglé pour délivrer une puissance de 60 Watts sous 0,5 Torr durant 6 minutes.

### Fonctionnalisation des lames d'ITO par le cyclooctyne 14

Les lames d'ITO ayant subi le traitement de surface décrit ci-dessus sont immergées dans une solution de 20 mg de cyclooctyne **14** dans 15 mL de toluène et placées à 100 °C pendant 20 heures dans l'étuve dans un système fermé. Elles sont ensuite rincées à l'eau déionisée et à l'acétone et séchées sous argon.

On obtient alors l'immobilisation de la molécule **14** sur la surface d'ITO via la formation d'interactions électrostatiques entre les atomes d'oxygène du motif carboxylate de **14** et les fonctions hydroxyles et/ou les cations métalliques de surface.

### Couplage du complexe 7

Les lames d'ITO ainsi fonctionnalisées sont alors immergées dans une solution de 6 mg de complexe **7** dissous dans un mélange de 4 ml d'eau distillée/méthanol (1/1) (cf. schéma). La solution est agitée pendant 12 heures. Les lames sont ensuite rincées à l'eau distillée et à l'acétone.

Les analyses XPS (spectroscopie de photoélectrons) de la surface obtenue confirment la présence de cobalt lequel est absent lorsque les surfaces sont analysées avant réaction avec **7.** Cette fonctionnalisation résiste aux lavages avec l'eau distillée et à l'ultrasonication.

### Exemple 7 : Immobilisation d'un complexe de coordination sur un tapis de nanotubes de carbone

Le schéma ci-dessous illustre les réactions effectuées dans le cadre de cet exemple.

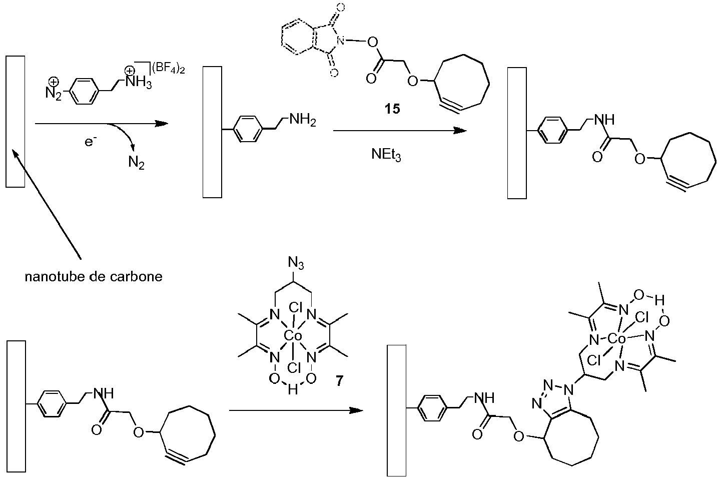

La procédure de fonctionnalisation passe, tout d'abord, par l'immobilisation de nanotubes de carbone sur une électrode de fibre de carbone recouverte d'un enduit conducteur microporeux (couche de diffusion gazeuse). Les nanotubes de carbone sont ensuite décorés par des fonctions amines.

Le complexe de cobalt **7** est, en dernier lieu, immobilisé sur le tapis de nanotubes de carbone décorés et déposés sur la couche de diffusion gazeuse.

### 1) Fonctionnalisation des nanotubes de carbone

Les couches de diffusion gazeuse (BASF LT1200W) sont fournies par BASF.

Les nanotubes de carbone multi-paroi C100 (> 95 %) ont été achetés chez Arkema et utilisés sans étape de purification supplémentaire.

Les électrodes recouvertes de nanotubes de carbone (NTCs/GDL 15 cm²; 0,1 mg_{NTCs.}cm⁻²) ont été réalisées à partir de couches de diffusion gazeuse (BASF) et fonctionnalisées par électro-réduction du sel de diazonium (4-(2-aminoethyl)benzenediazonium tetrafluoroborate).

Les électrodes recouvertes de nanotubes de carbone ont été utilisées comme électrodes de travail dans une cellule à trois électrodes en présence de 4-(2-aminoethyl)benzenediazonium tetrafluoroborate (1 mmol/L). Trois cycles de voltammétrie cyclique, menés à 20 mV/s et entre 0,4 V et -0,4 V vs Fc/Fc⁺, permettent d'observer à un potentiel de -0,26 V vs Fc/Fc⁺, 0,01 mol/L, la réduction du sel de diazonium aboutissant à la décoration des électrodes par des fonctions amines.

Les électrodes sont ensuite rincées avec de l'acétonitrile par trempage durant 30 mn puis séchées à l'air plusieurs heures avant utilisation.

### 2) Immobilisation sur les nanotubes du complexe de coordination

Dans un second temps, les fonctions amines précédemment obtenues sont amenées à réagir avec un cyclooctyne **15** fonctionnalisé par une fonction ester activée en présence de triéthylamine (cf. schéma). Le complexe **7** est ensuite immobilisé par un couplage de Huisgen et se révèle actif pour la réduction du proton en dihydrogène.

Le 1,3-dioxoisoindolin-2-yl 2-(cyclooct-2-ynyloxy)acetate **15** est synthétisé selon le protocole suivant : à une solution de 84 mg (1 eq.) de l'acide cyclooct-1-yn-3-glycolique **14** dans 15 ml d'acétate d'éthyle est ajouté 80 mg (1 eq.) N-Hydroxyphthalimide et 101 mg (1 eq.) de *N*,*N'*-Dicyclohexylcarbodiimide. Après 12 heures d'agitation à température ambiante, le mélange est filtré et le filtrat est purifié, après concentration, par colonne chromatographie (Cyclohexane / acétate d'éthyle 90/10). 143 mg de produit est récupéré sous la forme d'un solide blanc.
¹H NMR (300 MHz, CDCl₃) : 1,3-2,3 (m, 10H); 4,48 (m, 1H); 4,57 (d, j=17 Hz, 2H) ; 7,85 (m, 4H)
¹³C NMR (75 MHz, CDCl₃): 20,71 ; 26,09 ; 29,58 ; 34,23 ; 42,17 ; 55,74 ; 63,98 ; 90,78 ; 102,39; 124,04; 128,83 ; 134,87 ; 161,58 ; 166,77

Les couches de diffusion gazeuse recouvertes de nanotubes de carbone fonctionnalisés par des fonctions amine sont alors plongées pendant 2 jours dans une solution de **15** (10 mg) et Et₃N (10 µL, 3 mM) dans CH₂Cl₂ (25 mL). Les électrodes sont ensuite lavées par trempage dans une solution de CH₂Cl₂ et séchées à l'air.

Les électrodes sont alors plongées pendant une semaine dans une solution de 7 (5 mg) dans l'éthanol (25 mL) puis lavées par trois trempages successifs (30 min, 25 mL) dans de l'éthanol. Les électrodes ainsi obtenues sont enfin séchées à l'air.

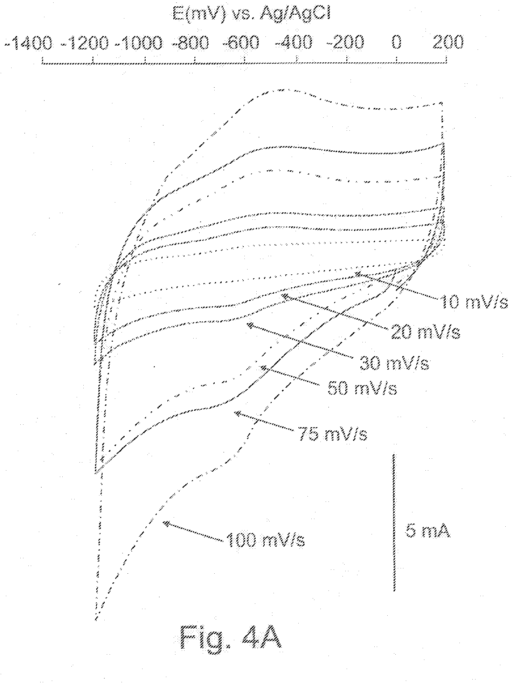

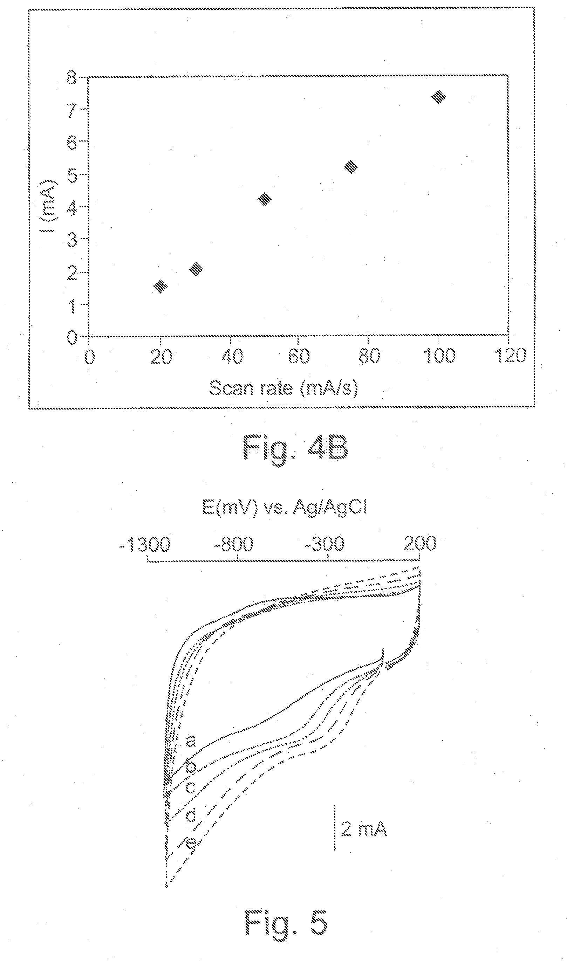

Les voltammogrammes cycliques, enregistrés dans l'acétonitrile, sur une électrode de nanotubes de carbone fonctionnalisés par le complexe diimine-dioxime de cobalt sont fournis à la figure 4A. La présence d'une vague électrochimique réversible à -600 mV vs Ag/AgCl est la signature du complexe diimine-dioxime de cobalt. L'évolution des courants de pics est proportionnelle à la vitesse de balayage (cf. figure 4B), ce qui est indicatif d'un motif électro-actif immobilisé (et non adsorbé) sur la surface de l'électrode de travail.

Le voltammogramme de la figure 5 présente l'évolution de ce voltammogramme lorsque du tétrafluoroborate d'anilinium est ajouté en solution. Cet acide est utilisé comme source de proton. On note que la vague électrochimique se déplace vers les potentiels positifs et augmente en intensité lorsque la quantité d'acide augmente. Les courbes a, b, c, d et e correspondent respectivement à des concentrations en anilinium égales à 0, 1,8, 2,8, 4,0 et 5,2 mmol.L⁻¹. Ceci est typique d'un comportement électrocatalytique de production de dihydrogène.

### Exemple 8 : Illustration d'une immobilisation du ligand 6 sur ITO pour la complexation de métaux

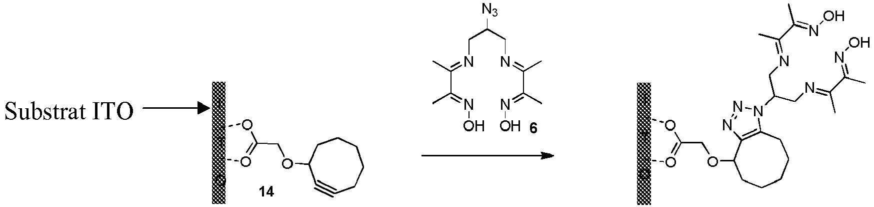

Le ligand **6** non complexé peut être greffé sur un oxyde selon un procédé analogue à celui donné dans l'exemple 7. Le matériau obtenu peut ensuite complexer un atome métallique (cuivre, cobalt, nickel...) présent dans une solution. Les applications peuvent concerner la formation de capteur électrochimique de métaux si le substrat est conducteur, de matériaux pour la dépollution d'effluents...

L'expression « comportant un(e) » doit être comprise comme « comportant au moins un(e) ».

## Revendications

1. Substrat en surface duquel est immobilisé au moins un motif de formule (I) : 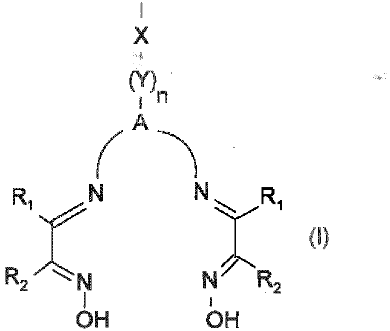 dans laquelle :
- R₁ et R₂, identiques ou différents, symbolisent un groupement choisi parmi : -H, un atome d'halogène, un éther en C₁ à C₂₀, une chaîne alkyle en C₁ à C₂₀, linéaire ou ramifiée éventuellement interrompue par un ou plusieurs hétéroatomes et/ou un ou plusieurs groupements aryles, ou un groupement aryle en C₅ à C₃₀ comprenant éventuellement un ou plusieurs hétéroatomes, ledit éther, ladite chaîne alkyle et ledit groupement aryle pouvant être substitués par au moins un des groupements suivants : -OH, -NH₂, -NO₂, -COOH, -CONH₂, un cycle triazole ou -COOZ₁ avec Z₁ représentant un groupe alkyle en C₁ à C₅, un groupe alcoxy en C₁ à C₅ ou un halogène,
- X est le produit d'une réaction de type « chimie-click », aza-Wittig, couplage peptidique ou le produit résultant de l'hydrogénation du produit obtenu à l'issue de ladite réaction d'aza-Wittig,
- Y est un bras espaceur,
- n est un nombre égal à 0 ou 1, et
- A:
- représente une chaîne hydrocarbonée, saturée ou insaturée, en C₂ à C₃, ou
- figure une double liaison C=C d'un cycle aromatique ou hétéroaromatique en C₅ à C₆ auquel cycle est lié Y, si n=1, ou X, si n=0,
une réaction de type « chimie-click » désignant une réaction de couplage entre une fonction alcyne et une fonction azoture.

2. Substrat selon la revendication 1 dans lequel X est de formule : 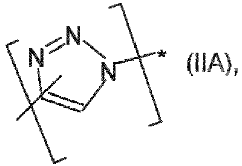 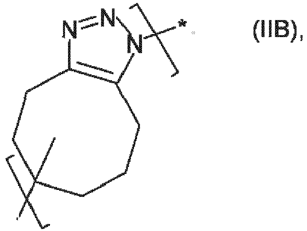 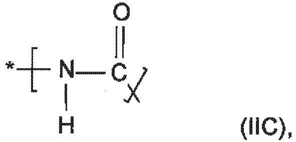 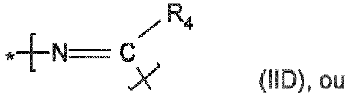 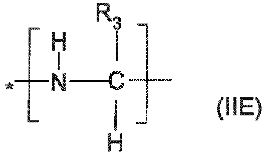 dans lesquelles :
- * symbolise une liaison vers Y si n=1 ou vers A si n=0, et
- R₃ et R₄ sont choisis parmi : -H, une chaîne alkyle en C₁ à C₂₀, un éther en C₁ à C₂₀, un ester en C₁ à C₂₀ ou un groupement aryle en C₅ à C₃₀, R₃ et R₄ étant le cas échéant substitués par au moins un des groupements suivants : -OH, -NH₂, -COOH ou -CONH₂.

3. Substrat selon l'une des revendications 1 et 2 dans lequel X est de formule : 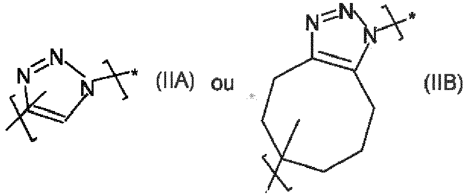 avec * étant tel que défini en revendication 2.

4. Substrat selon l'une quelconque des revendications précédentes dans lequel Y est choisi parmi une chaîne alkyle en C₁ à C₂₀, linéaire ou ramifiée éventuellement interrompue par un ou plusieurs hétéroatomes et/ou un ou plusieurs groupements aryles, un éther en C₁ à C₂₀, un ester en C₁ à C₂₀ ou un groupement aryle en C₅ à C₃₀ comprenant éventuellement un ou plusieurs hétéroatomes, Y étant le cas échéant substitué par au moins un des groupements suivants : -OH, -NH₂, -NO₂, -COOH, -CONH₂ ou -COOZ₂ avec Z₂ représentant un groupe alkyle en C₁ à C₅, un groupe alcoxy en C₁ à C₅ ou un halogène.

5. Substrat selon l'une quelconque des revendications 1 à 3 dans lequel le motif de formule (I) présente l'une des formules suivantes : 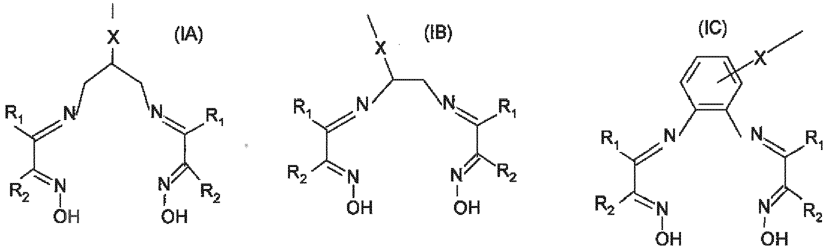 où R₁, R₂ sont tels que définis en revendication 1 et X est tel que défini selon l'une quelconque des revendications 1 à 3.

6. Substrat selon l'une quelconque des revendications précédentes dans lequel au moins un motif de structure (I) est coordiné à au moins un métal de manière à former au moins un complexe métallique (C).

7. Substrat selon la revendication précédente dans lequel le métal est un métal de transition de la classification périodique et plus particulièrement choisi parmi le nickel (Ni) et le cobalt (Co).

8. Substrat selon l'une des revendications 6 et 7 dans lequel le complexe métallique (C) formé avec ledit motif de formule (I) est comme suit: 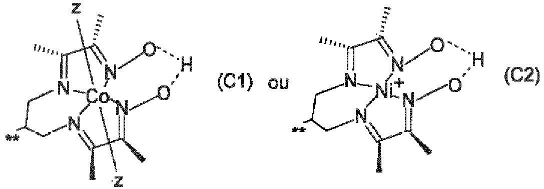 où Z désigne un atome d'halogène et ** symbolise la liaison vers X via Y si n=1.

9. Substrat selon l'une quelconque des revendications précédentes dans lequel le motif de formule (I) est lié au substrat via un bras de liaison.

10. Substrat selon la revendication précédente dans lequel le bras de liaison est lié à un ou plusieurs motifs de formule (I).

11. Substrat selon l'une des revendications 9 et 10 dans lequel le bras de liaison est lié au substrat par une interaction covalente ou non-covalente.

12. Substrat selon l'une quelconque des revendications précédentes **caractérisé en ce que**:
- il est formé en tout ou partie d'un matériau choisi parmi :
- les oxydes semi-conducteur par exemple ITO (« Indium Tin Oxide »), F:SnO₂ (« Fluorine Tin Oxide »), ZnO, Al:ZnO (ZnO dopé à l'aluminium), Na:ZnO (ZnO dopé au sodium), NiO ou TiO₂, ou
- les matériaux conducteurs ou semi-conducteurs par exemple :
- les métaux par exemple le silicium, éventuellement dopé, le laiton, l'acier inoxydable, le fer, le cuivre, le nickel, le cobalt, l'aluminium, l'argent, l'or, le titane,
- les oxydes métalliques, ou
- les matériaux carbonés par exemple les nanotubes de carbone mono ou multiparois, le noir de carbone, les nanoparticules fullereniques, le graphite, le carbone vitreux, le graphène ou le diamant dopé, ou
- il consiste en une plusieurs molécule(s) d'intérêt notamment une ou plusieurs molécule(s) biologique(s).

13. Electrode comportant un substrat selon l'une quelconque des revendications précédentes.

14. Dispositif d'électrolyse comportant une électrode selon la revendication précédente.

15. Ligand de formule (II): 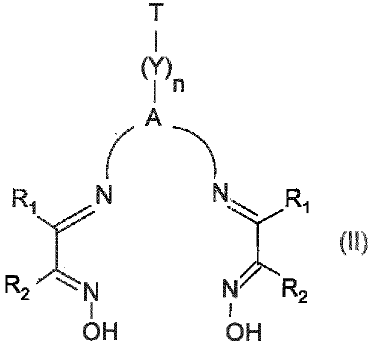 où:
- R₁, R₂, A et n sont tels que définis en revendication 1 et Y est tel que défini en revendications 1 ou 4, et
- T symbolise l'un des groupements suivants : N₃, NH₂ ou N=P(Ar)₃ pour lequel (Ar)₃ représente trois groupements aromatiques en C₆ à C₂₀, identiques ou non, éventuellement substitués, N=P(Ar)₃ étant de préférence N=PPh₃.

16. Ligand de formule (III): 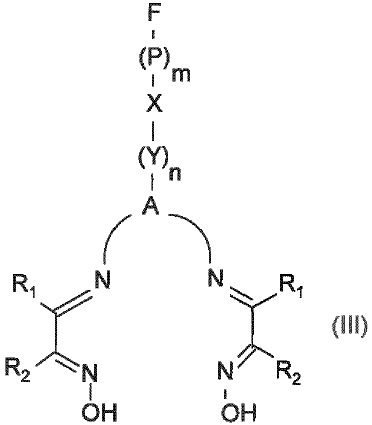 où :
- R₁, R₂, A et n sont tels que définis en revendication 1, Y est tel que défini en revendications 1 ou 4 et X est tel que défini dans l'une quelconque des revendications 1 à 3,
- m est un nombre égal à 0 ou 1,
- P symbolise une chaîne hydrocarbonée en C₁ à C₂₀ comprenant le cas échéant un ou plusieurs hétéroatomes, éventuellement interrompue par un ou plusieurs groupements aryles, un éther en C₁ à C₂₀, un ester en C₁ à C₂₀ ou un groupement aryle en C₅ à C₃₀ comprenant éventuellement un ou plusieurs atomes intracycliques, P peut le cas échéant être substitué par au moins un des groupements suivants : -OH, -NH₂, -NO₂, -COOH, -CONH₂ ou -COOZ₂ avec Z₂ représentant un groupe alkyle en C₁ à C₅, un groupe alcoxy en C₁ à C₅ ou un halogène, et
- F désigne une fonction réactive apte à réaliser une liaison covalente ou non covalente et en particulier choisi parmi -COOH, -NH₂, -N₃, -C≡CH et un groupe silane choisi parmi : un groupe trihalogénosilane, un groupe trihydrogénosilane, un groupe trialcoxysilane -Si(OR₅)₃- avec R₅ représentant un groupe alkyle de 1 à 6 atomes de carbone, linéaire ou ramifié, un groupe phényle, un groupe triaminoalcoxysilane -Si(NR₆R₇)₃, avec R₆ et R₇ représentant indépendamment l'un de l'autre un groupe alkyle saturé de 1 à 6 atomes de carbone, linéaire ou ramifié, un groupe phényle ou un groupe organométallique.

## Patentansprüche

1. Substrat, an dessen Oberfläche mindestens eine Einheit der Formel (I): 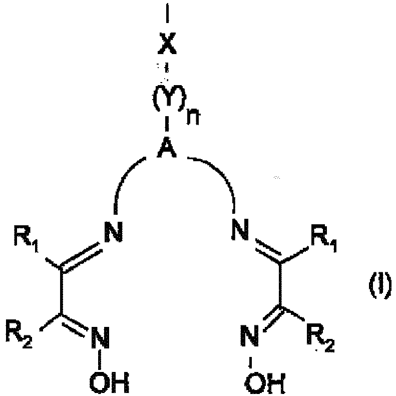 immobilisiert ist, wobei:
- R₁ und R₂ gleich oder verschieden sind und eine Gruppe symbolisieren, die aus -H, einem Halogenatom, einem C₁- bis C₂₀-Ether, einer linearen oder verzweigten C₁- bis C₂₀-Alkylkette, die gegebenenfalls durch ein oder mehrere Heteroatome und/oder eine oder mehrere Arylgruppen unterbrochen ist, oder einer C₅-bis C₃₀-Arylgruppe, die gegebenenfalls ein oder mehrere Heteroatome umfasst, ausgewählt ist, wobei der Ether, die Alkylkette und die Arylgruppe durch mindestens eine der folgenden Gruppen substituiert sein können: -OH, -NH₂, - NO₂, -COOH, -CONH₂, einen Triazolring oder -COOZ₁, wobei Z₁ für eine C₁- bis C₅-Alkylgruppe, eine C₁- bis C₅-Alkoxygruppe oder ein Halogen steht,
- X das Produkt einer Reaktion vom "Click-Chemie"-, Aza-Wittig- oder Peptidkupplungs-Typ oder das Produkt der Hydrierung des am Ende der Aza-Wittig-Reaktion erhaltenen Produkts ist,
- Y ein Spacer-Arm ist,
- n für eine Zahl mit einem Wert von 0 oder 1 steht und
- A:
- für eine gesättigte oder ungesättigte C₂-bis C₃-Kohlenwasserstoffkette steht oder
- eine C=C-Doppelbindung eines aromatischen oder heteroaromatischen C₅- bis C₆-Rings, an den im Fall von n = 1 Y oder im Fall von n = 0 X gebunden ist, darstellt,
wobei eine Reaktion vom "Click-Chemie"-Typ eine Kupplungsreaktion zwischen einer Alkinfunktion und einer Azidfunktion bezeichnet.

2. Substrat nach Anspruch 1, wobei X die Formel: 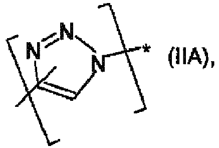 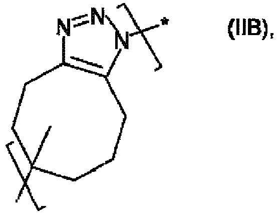 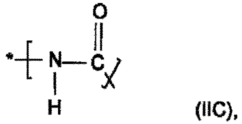 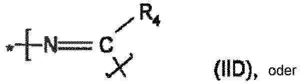 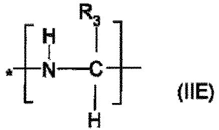 aufweist, wobei:
- * im Fall von n = 1 eine Bindung mit Y oder im Fall von n = 0 eine Bindung mit A symbolisiert und
- R₃ und R₄ aus: -H, einer C₁- bis C₂₀-Alkylkette, einem C₁- bis C₂₀-Ether, einem C₁- bis C₂₀-Ester oder einer C₅- bis C₃₀-Arylgruppe ausgewählt sind, wobei R₃ und R₄ gegebenenfalls durch mindestens eine der folgenden Gruppen substituiert sind: -OH, -NH₂, -COOH oder - CONH₂.

3. Substrat nach einem der Ansprüche 1 und 2, wobei X die Formel: 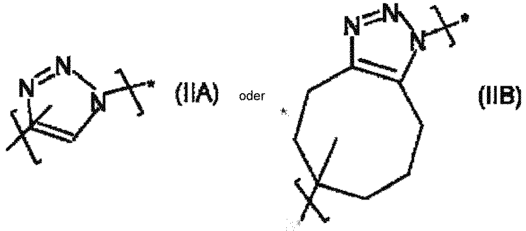 aufweist, wobei *wie in Anspruch 2 definiert ist.

4. Substrat nach einem der vorhergehenden Ansprüche, wobei Y aus einer linearen oder verzweigten C₁- bis C₂₀-Alkylkette, die gegebenenfalls durch ein oder mehrere Heteroatome und/oder eine oder mehrere Arylgruppen unterbrochen ist, einem C₁- bis C₂₀-Ether, einem C₁- bis C₂₀-Ester oder einer C₅- bis C₃₀-Arylgruppe, die gegebenenfalls ein oder mehrere Heteroatome umfasst, ausgewählt ist, wobei Y gegebenenfalls durch mindestens eine der folgenden Gruppen substituiert ist: -OH, -NH₂, - NO₂, -COOH, -CONH₂, einen Triazolring oder -COOZ₂, wobei Z₂ für eine C₁- bis C₅-Alkylgruppe, eine C₁-bis C₅-Alkoxygruppe oder ein Halogen steht.

5. Substrat nach einem der Ansprüche 1 bis 3, wobei die Einheit der Formel (I) eine der folgenden Formeln aufweist: 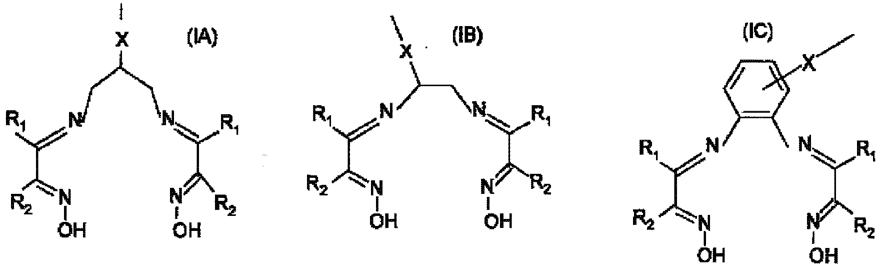 wobei R₁ und R₂ wie in Anspruch 1 definiert sind und X gemäß einem der Ansprüche 1 bis 3 definiert ist.

6. Substrat nach einem der vorhergehenden Ansprüche, wobei mindestens eine Einheit der Struktur (I) an mindestens ein Metall koordiniert ist, so dass sich mindestens ein Metallkomplex (C) ergibt.

7. Substrat nach dem vorhergehenden Anspruch, wobei es sich bei dem Metall um ein Übergangsmetall des Periodensystems handelt, das spezieller aus Nickel (Ni) und Cobalt (Co) ausgewählt ist.

8. Substrat nach einem der Ansprüche 6 und 7, wobei der mit der Einheit der Formel (I) gebildete Metallkomplex (C) wie folgt ist: 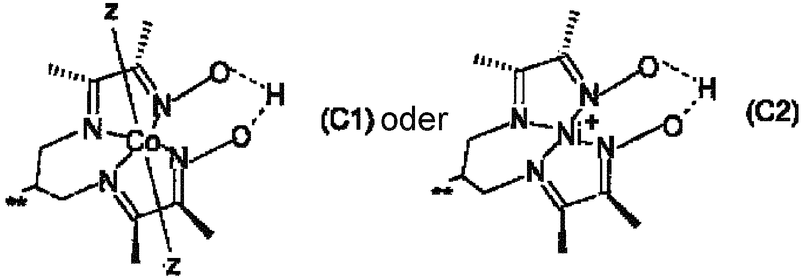 wobei Z für ein Halogenatom steht und ** die Bindung mit X über Y im Fall von n = 1 symbolisiert.

9. Substrat nach einem der vorhergehenden Ansprüche, wobei die Einheit der Formel (I) über einen Bindungsarm an das Substrat gebunden ist.

10. Substrat nach dem vorhergehenden Anspruch, wobei der Verbindungsarm an eine oder mehrere Einheiten der Formel (I) gebunden ist.

11. Substrat nach einem der Ansprüche 9 und 10, wobei der Verbindungsarm durch eine kovalente oder nichtkovalente Wechselwirkung an das Substrat gebunden ist.

12. Substrat nach einem der vorhergehenden Ansprüche, **dadurch gekennzeichnet, dass**:
- es ganz oder teilweise aus einem Material, das aus:
- halbleitenden Oxiden, beispielsweise ITO ("Indium Tin Oxide"), F:SnO₂ ("Fluorine Tin Oxide"), ZnO, Al:ZnO (mit Aluminium dotiertem ZnO), Na:ZnO (mit Natrium dotiertem ZnO), NiO oder TiO₂, oder
- leitenden oder halbleitenden Materialien, beispielsweise:
- Metallen, beispielsweise Silicium, gegebenenfalls dotiert, Messing, nichtrostendem Stahl, Eisen, Kupfer, Nickel, Cobalt, Aluminium, Silber, Gold, Titan,
- Metalloxiden oder
- Kohlenstoffmaterialien, beispielsweise ein- oder mehrwandigen Kohlenstoff-Nanoröhren, Ruß, Fulleren-Nanopartikeln, Graphit, glasartigem Kohlenstoff, Graphen oder dotiertem Diamant,
ausgewählt ist, gebildet ist oder
- es aus einem oder mehreren Molekülen von Interesse, insbesondere einem oder mehreren biologischen Molekülen, besteht.

13. Elektrode, umfassend ein Substrat nach einem der vorhergehenden Ansprüche.

14. Elektrolysevorrichtung, umfassend eine Elektrode nach dem vorhergehenden Anspruch.

15. Ligand der Formel (II): 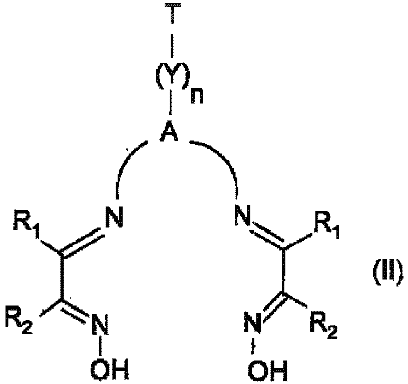 wobei:
- R₁, R₂, A und n wie in Anspruch 1 definiert sind und Y wie in Anspruch 1 oder 4 definiert ist und
- T eine der folgenden Gruppen symbolisiert: N₃, NH₂ oder N=P(Ar)₃, wobei (Ar)₃ für 3 gleiche oder verschiedene, gegebenenfalls substituierte aromatische C₆- bis C₂₀-Gruppen steht, wobei N=P(Ar)₃ vorzugsweise N=PPh₃ ist.

16. Ligand der Formel (III): 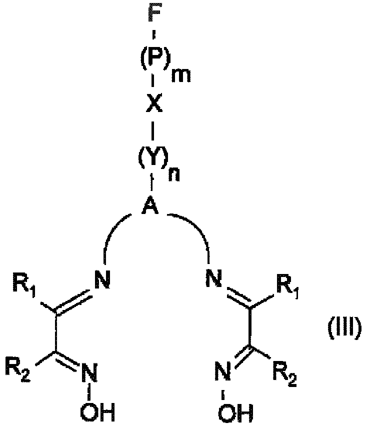 wobei:
- R₁, R₂, A und n wie in Anspruch 1 definiert sind, Y wie in Anspruch 1 oder 4 definiert ist und X wie in einem der Ansprüche 1 bis 3 definiert ist,
- m für eine Zahl mit einem Wert von 0 oder 1 steht,
- P eine C₁- bis C₂₀-Kohlenwasserstoffkette, die gegebenenfalls ein oder mehrere Heteroatome umfasst und gegebenenfalls durch eine oder mehrere Arylgruppen substituiert ist, einen C₁-bis C₂₀-Ether, C₁- bis C₂₀-Ester oder eine C₅-bis C₃₀-Arylgruppe, die gegebenenfalls ein oder mehrere intracyclische Atome umfasst, symbolisiert, wobei P gegebenenfalls durch mindestens eine der folgenden Gruppen substituiert ist: -OH, -NH₂, -NO₂, -COOH, -CONH₂ oder -COOZ₂, wobei Z₂ für eine C₁- bis C₅-Alkylgruppe, eine C₁- bis C₅-Alkoxygruppe oder ein Halogen steht, und
- F für eine reaktive Funktion steht, die eine kovalente oder nichtkovalente Bildung ausbilden kann und insbesondere aus - COOH, -NH₂, -N₃, -C=CH und einer Silangruppe, die aus einer Trihalogensilangruppe, einer Trihydrogensilangruppe, einer Trialkoxysilangruppe -Si(OR₅)₃, wobei R₅ für eine lineare oder verzweigte Alkylgruppe mit 1 bis 6 Kohlenstoffatomen oder eine Phenylgruppe steht, und einer Triaminoalkoxysilangruppe -Si(NR₆R₇)₃, wobei R₆ und R₇ unabhängig voneinander für eine lineare oder verzweigte gesättigte Alkylgruppe mit 1 bis 6 kohlenstoffatomen, eine Phenylgruppe oder eine metallorganische Gruppe stehen, ausgewählt ist, ausgewählt ist.

## Claims

1. Substrate, at the surface of which is immobilized at least one unit of formula (I): 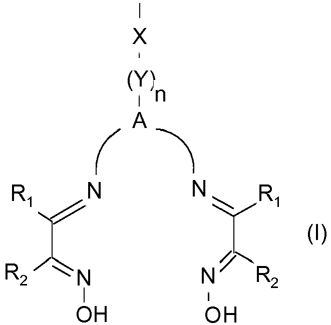 in which:
- R₁ and R₂, which may be identical or different, symbolize a group chosen from: -H, a halogen atom, a C₁ to C₂₀ ether, a linear or branched C₁ to C₂₀ alkyl chain optionally interrupted by one or more heteroatoms and/or one or more aryl groups, or a C₅ to C₃₀ aryl group optionally comprising one or more heteroatoms, the said ether, the said alkyl chain and the said aryl group being optionally substituted with at least one of the following groups: -OH, -NH₂, -NO₂, -COOH, -CONH₂, a triazole ring or -COOZ₁ with Z₁ representing a C₁ to C₅ alkyl group, a C₁ to C₅ alkoxy group or a halogen,
- X is the product of a reaction of "click-chemistry" type, aza-Wittig, peptide coupling or the product resulting from the hydrogenation of the product obtained after the said aza-Wittig reaction,
- Y is a spacer arm,
- n is a number equal to 0 or 1, and
- A:
- represents a saturated or unsaturated C₂ to C₃ hydrocarbon-based chain, or
- represents a double bond C=C of a C₅ to C₆ aromatic or heteroaromatic ring to which ring is attached Y, if n=1, or X, if n=0,
a reaction of "click-chemistry" type meaning a coupling reaction between an alkynyl function and an azide function.

2. Substrate according to claim 1, in which X is of formula: 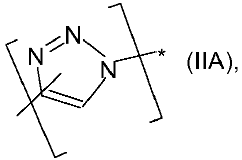 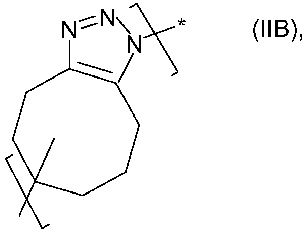 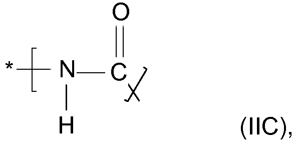 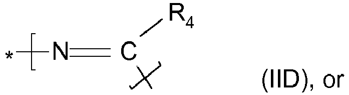 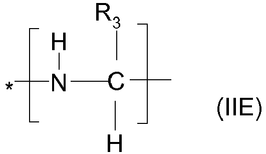 in which:
- * symbolizes a bond to Y if n=1 or to A if n=0, and
- R₃ and R₄ are chosen from: -H, a C₁ to C₂₀ alkyl chain, a C₁ to C₂₀ ether, a C₁ to C₂₀ ester or a C₅ to C₃₀ aryl group, R₃ and R₄ being optionally substituted with at least one of the following groups: -OH, -NH₂, -COOH or -CONH₂.

3. Substrate according to any one of claims 1 and 2, in which X is of formula: 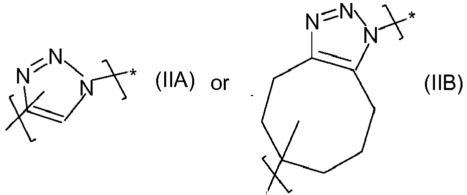 with * being as defined in claim 2.

4. Substrate according to any one of the preceding claims in which Y is chosen from a linear or branched C₁ to C₂₀ alkyl chain optionally interrupted by one or more heteroatoms and/or one or more aryl groups, a C₁ to C₂₀ ether, a C₁ to C₂₀ ester, or a C₅ to C₃₀ aryl group optionally comprising one or more heteroatoms, Y being optionally substituted with at least one of the following groups: -OH, -NH₂, -NO₂, -COOH, -CONH₂, or -COOZ₂ with Z₂ representing a C₁ to C₅ alkyl group, a C₁ to C₅ alkoxy group or a halogen.

5. Substrate according to any one of claims 1 to 3 in which the unit of formula (I) has one of the following formulae: 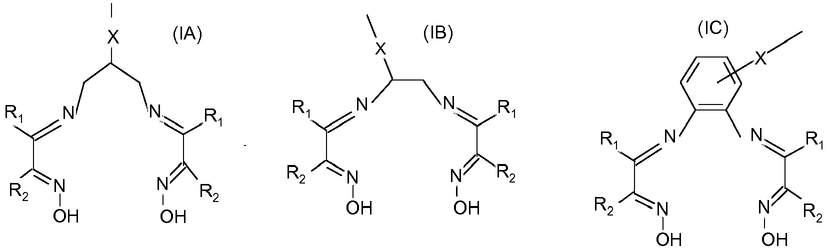 in which R₁ and R₂ are as defined in claim 1 and X is as defined in any one of claims 1 to 3.

6. Substrate according to any one of the preceding claims, in which at least one unit of structure (I) is coordinated to at least one metal so as to form at least one metal complex (C).

7. Substrate according to the preceding claim, in which the metal is a transition metal of the Periodic Table and more particularly chosen from nickel (Ni) and cobalt (Co).

8. Substrate according to any one of claims 6 and 7, in which the metal complex (C) formed with the said unit of formula (I) is as follows: 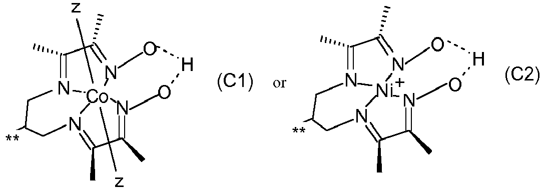 in which Z denotes a halogen atom and ** symbolizes the bond to X via Y if n=1.

9. Substrate according to any one of the preceding claims, in which the unit of formula (I) is bonded to the substrate via a linker arm.

10. Substrate according to the preceding claim, in which the linker arm is bonded to one or more units of formula (I).

11. Substrate according to any one of claims 9 and 10, in which the linker arm is bonded to the substrate via a covalent or non-covalent interaction.

12. Substrate according to any one of the preceding claims **characterized in that**:
- it is formed totally or partly from a material chosen from:
- semiconductive oxides, for example ITO ("Indium Tin Oxide"), F:SnO₂ ("Fluorine Tin Oxide"), ZnO, Al:ZnO (ZnO doped with aluminum), Na:ZnO (ZnO doped with sodium), NiO or TiO₂, or
- conductive or semiconductive materials for example:
- metals for example silicon, optionally doped, brass, stainless steel, iron, copper, nickel, cobalt, aluminum, silver, gold, titanium,
- metallic oxides, or
- carbon materials for example single-wall or multi-wall carbon nanotubes, carbon black, fullerenic nanoparticles, graphite, vitreous carbon graphene or doped diamond, or
- it consists of one or more molecules of interest, notably one or more biological material(s).

13. Electrode comprising a substrate according to any one of the preceding claims.

14. Electrolysis device comprising an electrode according to the preceding claim.

15. Ligand of formula (II): 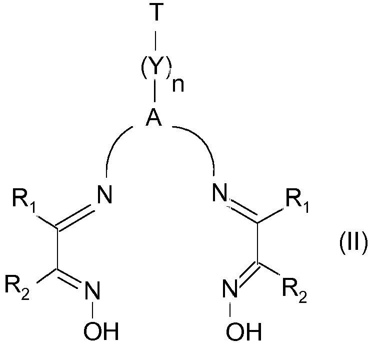 in which:
- R₁, R₂, A, and n are as defined in claim 1 and Y is as defined in claims 1 or 4, and
- T symbolizes one of the following groups: N₃, NH₂ or N=P(Ar)₃ for which (Ar)₃ represents three identical or different C₆ to C₂₀ aromatic groups, optionally substituted, N=P(Ar)₃ being preferably N=PPh₃.

16. Ligand of formula (III): 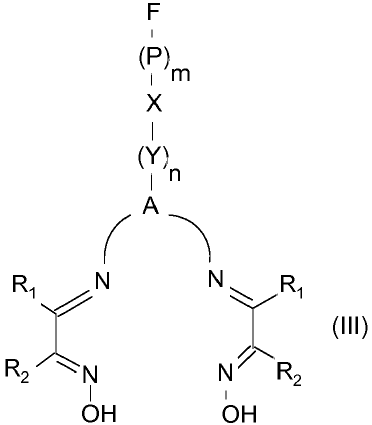 in which:
- R₁, R₂, A and n are as defined in claim 1, Y is as defined in claims 1 or 4 and X is as defined in any one of claims 1 to 3,
- m is a number equal to 0 or 1,
- P symbolizes a C₁ to C₂₀ hydrocarbon chain optionally comprising one or more heteroatoms, optionally interrupted by one or more aryl groups, a C₁ to C₂₀ ether, a C₁ to C₂₀ ester, or a C₅ to C₃₀ aryl group optionally comprising one or more intracyclic atoms, P being optionally substituted with at least one of the following groups: -OH, -NH₂, -NO₂, -COOH, -CONH₂, or -COOZ₂ with Z₂ representing a C₁ to C₅ alkyl group, a C₁ to C₅ alkoxy group or a halogen, and
- F denotes a reactive function that is capable of forming a covalent or non-covalent bond and in particular chosen from -COOH, -NH₂, -N₃, -C=CH and a silane group chosen from: a trihalogenosilane group, a trihydrogenosilane group, a -Si(OR₅)₃- trialkoxysilane group with R₅ representing a linear or branched alkyl group with 1 to 6 carbon atoms, a phenyl group, a -Si(NR₆R₇)₃ triaminoalkoxysilane group, with R₆ and R₇, independently of each other, representing a linear or branched saturated alkyl group with 1 to 6 carbon atoms, a phenyl group or an organometallic group.
